# EUROPÄISCHE PATENTANMELDUNG

(11) **EP 0 800 841 A1**
(43) Veröffentlichungstag der Anmeldung: **15.10.1997**
(21) Anmeldenummer: 96250064.1
(22) Anmeldetag: 16.03.1996
(51) Int. Cl.: A61M 5/30

(54) **Druckstrahlinjektor**

(71) Anmelder: Wagner, Wolfgang, Dr.med., 13503 Berlin (DE)
(72) Erfinder: Wagner, Wolfgang, Dr.med., 13503 Berlin (DE)

(57) **Zusammenfassung**

Es handelt sich um einen Injektor vor allem für die Behandlung insulinpflichtiger Diabetiker, aber auch zur chronischen Schmerzbekämpfung und Gerinnungsprophylaxe und anderen Zwecken unter Anwendung der nadellosen Druckstrahlinjektion in eine in einer Saugglocke angehobenen Haut, wobei der Einspritzkanal noch während der Arzneiinjektion, soweit es sich um eine Flüssigkeit handelt, mit Verdünnerflüssigkeit ausgewaschen wird.
Bei pulverförmiger Arznei wird die Funktion des Verdünners, aber auch des Wassers zur Düsenreinigung zwischen den Anwendungen, durch das Treibgas ersetzt.
Vorkehrungen zur optischen Hautüberwachung zum Ausschluß der Punktion krankhaften Gewebes und zur Wahrung der Keimfreiheit werden verbessert.

## Beschreibung

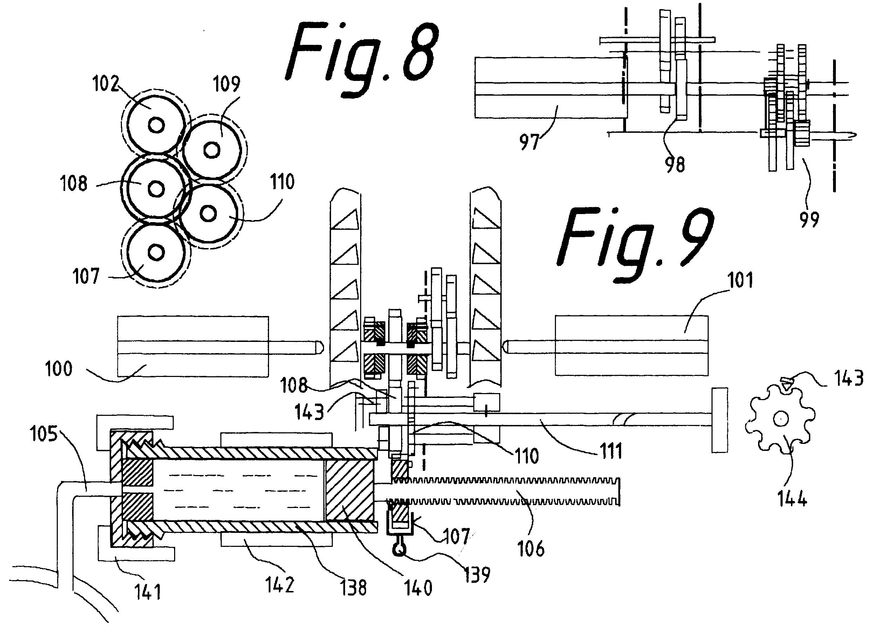

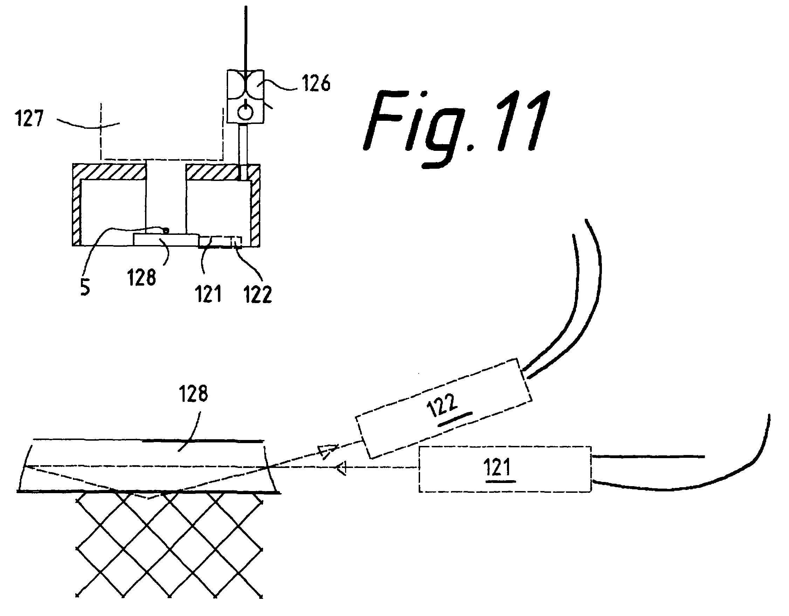

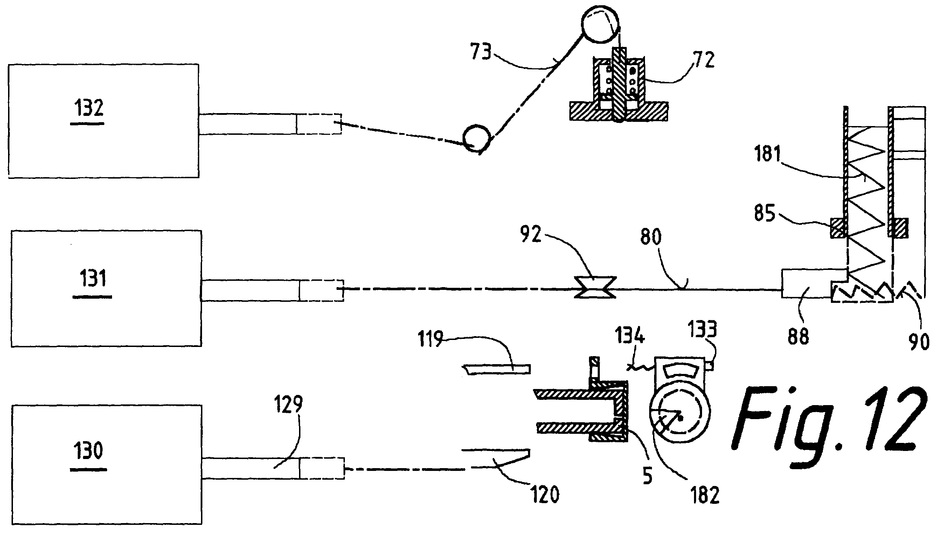

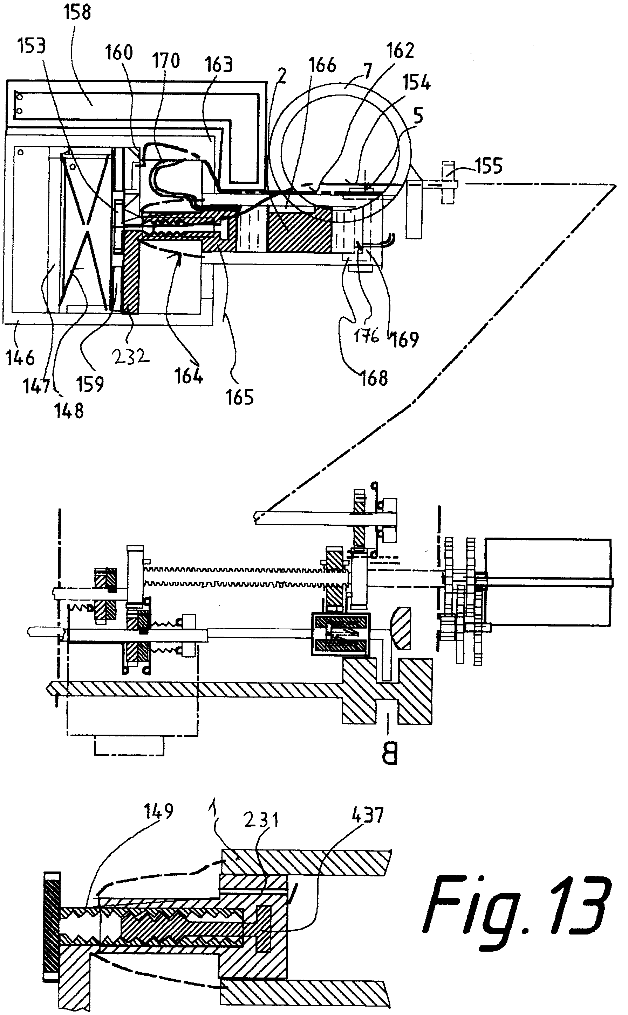

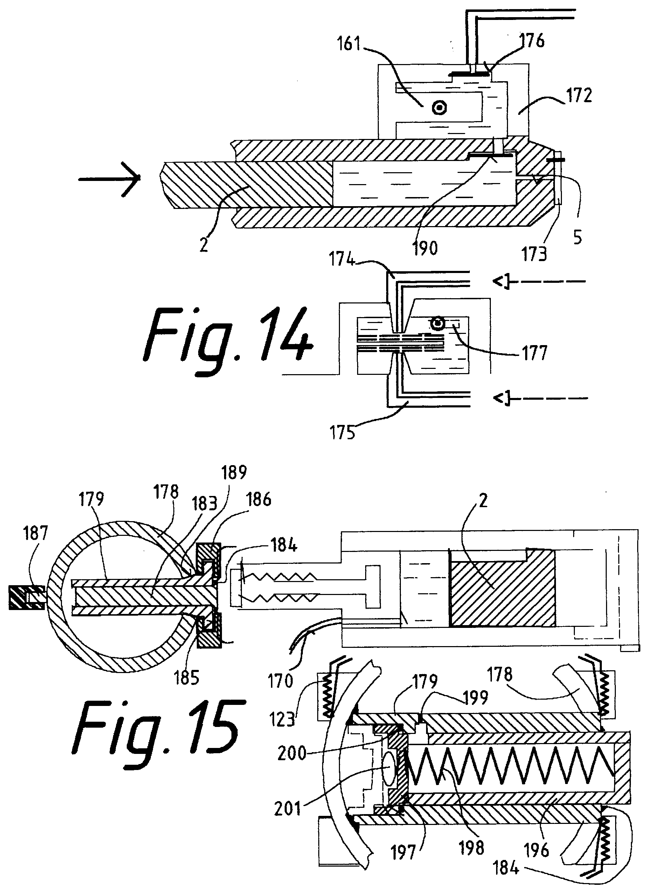

Die Erfindung bezieht sich auf die Medizintechnik, spezieller auf die Injektionstechnik, insbesondere aber auf die Behandlung von Zuckerkranken mit Insulin.
Einmal stützt sich die Erfindung auf die in den 70er Jahren aufkommende Überdruckstrahlinjektion, die sich bis 1983 noch bei der Reihenimpfpraxis halten konnte. Die bei wiederholter Anwendung auftretenden Hautreizungen waren teilweise auf Gewebszerreißungen zurückzuführen, da die Injektordüse gewebekomprimierend gegen die Haut gedrückt wurde. Diesem Mangel wurde in der Anmeldung P 37 30 469.0 durch Anhebung der Haut zu deren Verdünnung und zur Raumschaffung abgeholfen.
Die jetzige Anmeldung soll einen weiteren Grund für heftige Hautreizungen beseitigen: nämlich das Zurückbleiben von Arznei- und derselben beigefügter Desinfektionsmittelreste im Injektionskanal in der Haut. Außerdem soll die Injektuionsmenge durch Verdünnung kleinster Arzneimengen vergrössert und die automatische Düsenreinigung gewährleistet werden.
Die gestellte Aufgabe wird in einem Beispiel dadurch gelöst, daß zwei getrennte Kolben-Zylinder-Pumpen durch eine gemeinsame Düse entleert werden. Über einen elektrischen Kontaktschluß zwischen Kolbenstange und Zylinder kurz vor der Entleerung des ersten Zylinders mit der Arznei wird der Druckgeber -hier Elektromagnet oder Druckfeder- für den zweiten Kolben im Zylinder mit der gewebsfreundlichen Flüssigkeit gestartet; so daß der Druckstrahl nicht unterbrochen wird. Die Aufgabe kann auch durch einen Rechner gelöst werden, der sich an der in den ersten Zylinder eingeführten Arzneimenge orientiert. In dem bevorzugten Beispiel erreicht die vom Druckgeber bewegte Stange zuerst eine Kolbenschräge über dem Arzneizylinder und etwas später eine Keilschräge über dem zweiten Injektionszylinder mit gewebsfreundlicher Flüssigkeit oder Verdünner (etwa physiologischer Kochsalzlösung). Verdünner sagen wir, weil letztgenannte Flüssigkeit auch zur Verdünnung kleinster Arzneimengen im Injektionszylinder für Arznei (Arzneizylinder) dient.
Zur Dosierung wird auf die Stufenspritze (das "Pen-System") nur beispielsweise zurückgegriffen, um keine jetzt handelsunübliche Umwege zu gehen. In der angestrebten vollautomatisch, elektronisch gesteuerten Version, wird anstelle des Handdruckknopfes die Rotation der den Gummikolben in der Arzneipatrone vorschiebenden Mikrometerschraube mittels Motorantriebes angewandt(Beispiel Fig.8,9).
Über der Einspritzdüse ist eine Art Deckel und ein Mechanismus zu dessen Öffnung vorgesehen. Günstigerweise liegt dieser Deckel blendenartig als eine Art Blattfeder der Oberfläche des Endes des Injektionszylindes an(vgl.Fig.12 unten).
Diese Blende kann nun in vertikaler oder radialer Bewegung aus dem Bereich der Einspritzedüse mittels einer Keilschrägen durch einen Hebel oder direkt -etwa durch einen Bowdenzug oder Elektromagneten- verschoben werden.
Der Düsenstrahl kann auch schräg innerhalb der Hautkuppe geführt werden, kommt es doch auf eine Verlängerung der Wegstrecke an, um die Wucht des Druckstrahles zu brechen. Vorzugsweise wird für denselben Zweck die Düse so gestaltet, daß der Strahl in einem Knotenpunkt gebündelt in die Haut eintritt, um dann keilförmig sich zu verbreitern. Im Grenzfall erfolgt die Injektion beinahe waagerecht vom Saugglockenrand aus; hier tritt besonders inniger und sicherer Hautkontakt ein (berechenbare Düsendistanz!);
auch leichte Druckschwankungen können diesem Hautkontakt hier nichts anhaben.
Für die Sauberhaltung des Düsenbereiches unter Aufheizung kann eine Art zweite Blende über der eben beschriebenen angeordnet werden, welche die Heizdrähte enthält. Der Vorteil einer solchen Funktionsteilung ist der, daß die zweite Blende oder Abdeckung dicker gestaltet und noch vor Hochziehen der Haut aus dem Funktionsbereich herausgedreht oder gehoben werden kann.
Die optische Überwachung der Einspritzstelle in der Haut kann bereits in der Anhebungsphase der Haut geschehen, insbesondere wenn die Rechnerkapazität der elektrischen und elektronischen Steuereinheit höher ausgelegt ist. Es kann dann die natürliche Hautfelderung als eine Art Maßstab benutzt und die Reflexionsbeschaffenheit (Helligkeitswiedergabe) der Hautstelle, wie sie etwa an einem Fenster am Saugglockenrand ermittelt wurde, auf die vor der Düse liegende Stelle umgerechnet werden(vgl.Fig.11). Bei derartiger Rechnererfassung einer Hautfläche mit mehreren Bildpunkten, kann auch ein gesprengeltes Hautkolorit (etwa bei Sommersprossen) besser berücksichtigt werden. Zu derartiger Umrechnung wird die Dauer und Geschwindigkeit der Hautmusterbewegung herangezogen. Über elektronische Steuerung einer Belüftungsdrossel für die Saugglocke kann sogar eine pigmentarme Zone der Haut für die Injektion "angesteuert" werden, ohne den Vorgang der Hautansaugung vorher abbrechen und wiederholen zu müssen. Die Mindestanforderung an eine optische Hautkontrolle wird durch den Helligkeitsvergleich der Injektionsstelle mit einer benachbarten erfüllt; zweckmäßigweise noch unter Einbeziehung von Meßwerten aus Messungen bei vorhergehender Geräteanwendung. Bei einer vorteilhaften diaphanischen Lösung erfolgt die Messung durch eine Lochblende direkt vor der Düse tangential durch die dort eindringende kleine Hautblase. Werden "Meßkurven" während des Eindringens der Haut in die Lochblende ausgewertet, so kann die Kontrolle dadurch wesentlich verfeinert werden.
(Grobe Hauterhebungen treten erst gar nicht in die Lochblende ein).
Abgesehen von einer Blende zum Schutz der Düse vor Verschmutzung zwischen den Anwendungen, kann eine Blende -etwa zur Vermeidung von Ansaugung von Arznei vor der Injektion- entfallen, wenn hinter der Düse -etwa im Bereich des gemeinsamen Ausflußtrichters für die Injektionszylinder oder am Frontende des Injektionszylinders für Verdünnereine Ventilscheibe rotiert, welche die Düse annähernd zeitgleich mit der Aktivierung des Druckgebers für die Injektion über eine Aushöhlung freigibt. Parallel geschaltete Ventilscheiben in beiden Injektionszylindern steuern zweckmäßigerweise auch den Flüssigkeitsaustausch zwischen den Injektionszylindern. Auch bei einem Einzylindersystem kann der Zufluß von Arznei, Verdünner und Wasser jeweils über die Ventilscheibe gesteuert werden.
Ein Wasserbehälter war vorzusehen, da die Dosierzylinderkolben und Dosierzylinder wie auch die Düse von den Salzen der Verdünnerflüssigkeit (kurz: des Verdünners) nach jeder Injektion gereinigt werden müssen. Zweckmäßigerweise werden Verdünner und Wasser in hochelastischen Behältern gelagert und in eine Austausch-Behälter-Einheit zusammengefaßt. Da auch hier keinerlei Luft auch während des Behälterwechsels in das geschlossene Flüssigkeitssystem eindringen darf, müssen diese elastischen Behälter innerhalb eines weiteren Behälters mit starren Wandungen gelagert und in diesem ausgetauscht werden. Zweckmäßigerweise werden die Innenbehälter in einem weiteren Gerätebehältnis unter Gasdruck gesetzt.
Dies darf nur für die Zeit der Füllung des oder der Injjektionszylinder geschehen; jedenfalls erscheint eine solche zeitliche Beschränkung als zweckmäßig, um die Ventilausstattung einfach zu halten. Eine bevorzugte Ausgestaltung der Verdänner- Wasserbehälter besteht auf zwei Faltenbälge, die innerhalb eines Kunststoff- oder Pappzylinders von dessen Zwischenwand abgetrennt sind. Im Gerätebehälter erfolgt eine Trennung auch des Gasraumes um die beiden Faltenbälge. Hierzu wird beim Deckelschluß eine Art überstehender Kragenrand in der Mitte des Pappzylinders gegen eine Behälterdichtung gepreßt. Die zwei Zufuhrstutzen für Gas sind fest in der Gerätebehälterwand installiert. Die Ableitung von Verdünner bzw.Wasser erfolgt aus den Faltenbälgen über schlauchangeschlossene Kanülen. Letztere dringen vom Zentrum der aufschraubbaren Deckelkappe und vom Zentrum des Bodens des aufnehmenden Gerätebehälters her durch die Stopfenmembran im Zentrum jeweils des nach außen zeigenden Bodenteiles der beiden Faltenbälge. Bei der bevorzugten Flüssigkeitssteuerung allein durch die Ventilsscheiben in (oder um) die Injektionszylinder können die Faltenbälge auch ständig unter Gasdruck stehen. Mit dem Netzladegerät für Batterien ist zweckmäßigerweise eine Nachfüllvorrichtung für Wasser und Verdünner Verbunden, wobei auch Tagesvorräte an Wasser und Verdünner im Kunststoff-Pappzylinder ausgetauscht werden können.
Die Absicherung des oder der Injektionszylinder auf der Seite der Kolbeneinführung gegen Schmutz erfolgt durch Membran- oder Faltenbalgüberdachung. Neu ist die Einfüllung von Wasser oder anderer Flüssigkeit in den Raum zwischen Schutzmembran oder Faltenbalg und Kolbenhinterfläche. Dieses Vorgehen trägt zur Sicherung gegen das Eindringen von Luft in den Injektionsraum bei. Um bei kleinsten Arzneidosen auch die Hilfsflüssigkeit- und Wassermengen verringern zu können, wird auch für sie eine Dosisbegrenzung eingeführt. Besonders vorteilhaft kann hierfür die Hubhöhe der Injektionszylinderkolben (oder wenigstens des Kolbens für Verdünner) regelbar mechanisch begrenzt werden. Der Rechner kann gemäß solcher Beschränkung auf kleinere Arzneidosen dann kürzere Warnzeiträume für die Wiederbenutzung des Injektors festlegen.
Beim Einzylindersystem wird in einer Version das Arznei-Verdünner-Gemisch hinter der Düse durch einen (vorderen) Kolben vom Raum mit Verdünner abgetrennt, der von einem zweiten (hinteren) Kolben düsenwärts bewegt wird. Der vordere Kolben weist eine Ventilvorrichtung auf (zentral oder am Rande etwa durch Aussparungen, die mit ebensolchen in der Zylinderwand zur Deckung gebracht werden). Durch diese Ventilvorrichtung kann der Verdünner austreten, wenn die Kammer mit der Arznei nahezu entleert ist. Die Verdünnerzufuhr in die hintere Kammer erfolgt in der Regel über einen Schlauch durch den hinteren Kolben hindurch. Der hintere Kolben und die Ventilvorrichtung in vorderen lassen sich einsparen, wenn der Injektionszylinder besonders schmal gewählt wird und dessen Füllung unmittelbar vor der Injektion erfolgt(Fig.13).
Beispielsweise wird dann zunächst in Düsennähe Verdünner eingefüllt, dann eventuell eine Luftblase aus vorher erhitzter Kammer und schließlich die Arznei. Die sofort anschließende Injektion verhindert eine zu starke Durchmischung von Arznei und Verdünner bis zur Strahlerzeugung (Fig.14).
Als Druckgeber für die Injektionszylinderkolben können Druckgas, von einem Elektromotor über Getriebe gespannte Federn und/oder komprimierte Gaspolster (auch nacherhitzte) dienen.
Zur unterdruckerzeugung in der Saugglocke wird im Falle der Druckgasanwendung aus einer Vorratskapsel die Verwendung einer Gasstrahlpumpe möglich. Zur Herabsetzung des Aufwandes kann auch manueller Druck auf einen hinter einem gesonderten Deckel liegenden Beutel gegen in Rohrstutzen im Gehäuse -etwa an dessen vier Ecken- geführte Druckfedern ausgeübt werden. Der entstehende Überdruck wird über ein Ventil dem Behälter für die Faltenbälge (oder anders sich verkleinernden Behältern) mit Verdünner und Wasser zugeleitet. Wird der Flüssigkeitszustrom in den oder die Injektionszylinder geregelt (wie durch das erwähnte düsennahe Ventil, vorzugsweise als im Zylinder gelagertes Scheibenventil), so kann bei Einsatz eines Überdruckventils der Deckel der Deckplatte des Gerätes -dem handlichen Transport entgegenkommend- bis zur Verrasterung genähert werden.
Da der Beutel großflächig sowohl mit dem Deckel als auch der Deckplatte des Gerätegehäuses verbunden ist, erzeugt seine Wiederausdehnung nach Rasterauslösung der Druckfedern einen Sog, der über ein Rückschlagventil in der Saugglocke wirksam wird.

Entsprechend der Hauptaufgabe der Erfindung eines taschengängigen Injektors war der Vorrat an Flüssigkeit möglichst klein zu halten, vorallem der Vorrat an Verdünner und Wasser für einen Druckstrahlinjektor.
Es wird deshalb vorgeschlagen, den Injektor jeweils nur mit einer maximalen Tagesdosis benötigter solcher raumfordernder Flüssigkeiten zu versehen und die Bevorratung in einem Versorgungsgerät, welches mit einer Vielzahl kleiner Tages-Einzeldosen beschickt wird. Vorzugsweise ist dieses Versorgungsgerät zugleich das Ladegerät für die Batterien des Injektors und kann auch Rechnerkapazitäten und Registriervorrichtung umfassen.

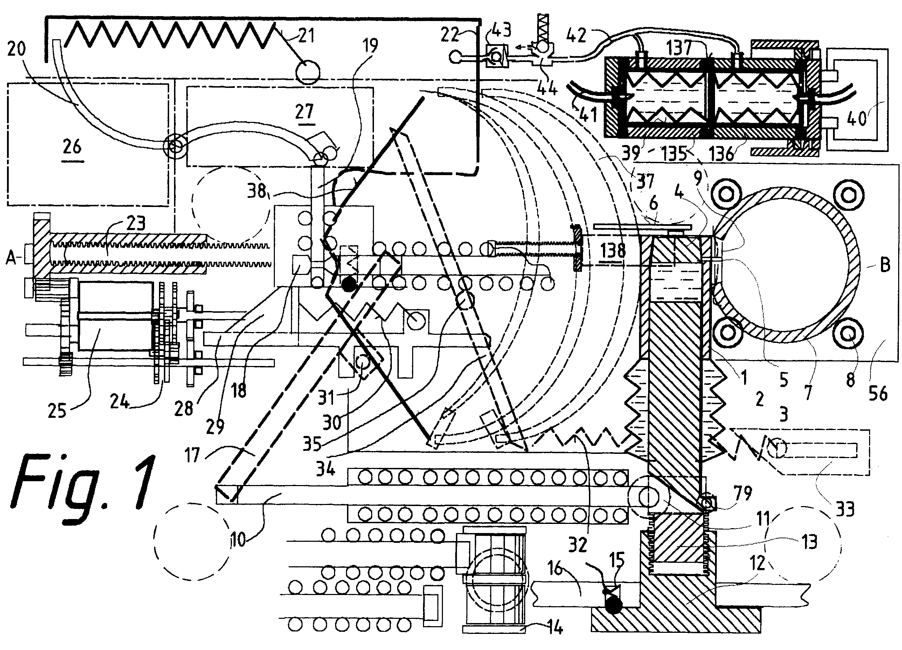

Es könnten zur Lösung dieser Aufgabe verkleinerte Vorrats-Pappzylinder(39,Fig.1) verwendet werden, die Ankoppelung wäre jedoch aufwendig. Es wird deshalb vorgeschlagen, sowohl Verdünner- als auch Wasserbehälter auf derselber Seite zu entleeren. Die Behälter können dabei, etwa als Faltenbälge, übereinander angeordnet sein, aber auch nebeneinander. Sie sind zweckmäßigerweise wieder je zu einer Einheit zusammengefaßt. Die Verbindung zu einer im Versorgungsgerät umlaufenden Kette von Einheiten erleichtert die Nachfüllung. Hierzu können Vorratsbehälter von einer Art Rahmenplatte am dem Austrittsstutzen abgewandten Ende eingefaßt sein. Sie können auch auf der Seite der Austritts-oder Abfüllstutzen -etwa auch entlang derselben- mit Klebeband verbunden und so in wiederverwendete Zylinder des Versorgungsgerätes eingeschoben werden. (Das Klebeband wird dann abgezogen). Der oder die Austrittsstutzen werden injektorseits zweckmäßig in eine Kammer eingeführt, die zeitweilig zu Zwecken der Sterilisation beheizt werden kann. Der Transport der sehen werden, der auch bei der Bewegung zur Trennung des Injektors vom Versorgungsgerät gegen eine Feder gespannt werden kann, aber dessen Mitnehmerklinke zusammen mit der vorgenannten Feder ausgelöst wird. Das entstehende Bewegungsmoment der Mitnehmerklinken ist entgegengesetzt bezogen auf das. Gerät, aber synergistisch auf die Behälterkette. Auch die zwei, vier oder mehr Kettenräder könnten einen Motorantrieb erhalten.
Da der Injektor vorzugsweise flach gestaltet werden soll, empfiehlt sich, die Schienenführung zwischen Injektor und Versorgungsgerät so anzuordnen, daß die Behälter jeweils liegend also parallel zur Längsachse des Injektors in diesen eingebracht werden können. Der Injektor kann dazu hochkant um 90 Winkelgrade gekippt auf das Versorgungsgerät aufgeschoben werden. Die Koppelung kann natürlich auch seitzuseit erfolgen bei Kippung der Kettenachsen im Versorgunggerät um einen rechten Winkel oder es werden die Behältereinheiten drehen um ihre Bodenflächen, die in der Kette gelagert oder sternförmig in einem Rad angeordnet sind. Schließlich kann natürlich auch eine Wiederauffüllung kleiner Dauerbehälter für Verdünner und Wasser im Injektor aus großen Behältern im Versorgungsgerät erfolgen, wenn die Vorkehrungen für eine luft- und keimfreie Koppelung beachtet werden, wie sie bereits in DE 25 51 991 A1, DE 25 51 992 A1, DE 25 51 993 A1 und WO 86/01728 (PCT/DE85/00313) analog behandelt wurde.
Als Neuerung wird jedoch die Dosiervorrichtung für den Nachfüllvorgang vom Injektor in das Versorgungsgerät verlegt.
Was den Injektor anlangt, so ist auch er eine Fortentwicklung und Anpassung an die Aufgaben und Lösungen, wie sie in PCT/DE85/00113 (Euro 0 221005 veröffentlicht am 6.5.87) aufgezeigt wurden. Dies betrifft die Programmierung sowohl als auch die Koppelung mit einem Meßwertaufnehmer für Glukose, se, wie er als Infrarotlaser die Blutleere in der Saugglocke nutzen kann (aber auch andere chemische Methoden). Auch kabelfreie Datenübertragung auf und vom Injektor sind möglich sowie dessen Nutzung etwa bei der Heparinisierung zur Thromboseprophylaxe und zur Schmerzbekämpfung.

### Kurze Beschreibung der Ausführungsbeispiele:

Die Figur 1 zeigt in natürlicher Größe einen schematischen Längsschnitt die bevorzugte Lösung des Injektors, wobei die Gehäusewandung nur in einem Bruchstück angedeutet wurde. Die Funktionsteile wurden der Übersichtlichkeit wegen nebeneinander aufgereiht. Unten in der Mitte werden in einer Seitenansicht die beiden Kantstifte als Druckgeber den beiden Anschrägungen der Injektionskolben zugeordnet.
Die Figur 2 gibt eine Seitenansicht eines Schaltgetriebe-Details in Bowden-Kabelverbindung mit den beiden Ventilscheiben der Injektionszylinder (rechts) in natürlicher Größe. Links der an die Steuerung angeschlossene Injektionszylinder in einer anderen Ventilstellung, in der Mitte im Maßstab 2 : 1 ein Vertikalschnitt durch die beiEnden der beiden Injektionszylinder mit den Ventilscheiben. Links unten das Detail einen um einen Injektionszylinder kreisenden Ventilkappe als Alternative zur Ventilscheibe im Vertikalschnitt in natürlicher Größe.
Die Figur 3 zeigt oben im Querschnitt längs der Schnittlinie A - B des Längsschnittes der Fig.1 weitere Funktionsmechanismen und der mechanischen Grundlagen in natürlicher Größe und zwei Funktionsstadien untereinander.
Die Figur 4 zeiugt oben in einem Längsschnitt in natürlicher Größe des Detail eines Injektionszylinders in Nähe der Saugglocke, darunter im Querschnitt längs der Schnittlinie A - B des Längsschnittes im Maßstab 2 : 1 einen Teil der Saugglocke mit einem Auslöserstift für den Sog.
Die Figur 5 zeigt in einem Längsschnitt durch den Schieber mit den Fenster eine Vorrichtung zu optischen Hautkontrolle im Maßstab 4 : 1 .
Die Figur 6 zeigt im Vertikalschnitt im Maßstab 2 : 1 die Befestigung des Schiebedeckels für den Saugglockenverschluß an einer Gehäusewand.

Die Figur 7 zeigt in einem Querschnitt längs eines seitlichen Injektorteiles (nahe einer Seitenwand) schematisierend den Mechanismus zur Druck- und Sogerzeugung im Deckelbereich, links im entfalteten Zustand des Beutels, rechts im Zustand der Druckfederverrasterung entsprechend der Beutelentleerung. Als Details im Maßstab 2 : 1 Profile an der Beuteloberfläche für dessen flächige Befestigung.
Die Figur 8 im Längsschnitt in natürlicher Größe eine Lösung für den Antrieb eines Teiles des mechanischen Steuergetriebes durch einen kleinen Elektromotor.
Die Figur 9 zeigt ein Steuergetriebe für die Arzneidosierung in Verbindung mit der Arzneipatrone im Längsschnitt im Maßstab 2 : 1. Rechts ein Vertikalschnitt durch eine der Rasterscheiben für die Dosierstufenabgrenzung, links oben ein Vertikalschnitt zur Verdeutlichung des Umschaltgetriebes für die Bewegungsumkehr nach Verbrauch der Arzneipatrone.
Die Figur 10 zeigt im Maßstab 2 : 1 eine Vorrichtung zur Düsenabdeckung und zur optischen Hautkontrolle in einer Saugglocke als Alternativlösung und zwar oben im Querschnitt, links unten in einer Aufsicht (von unten her).
Rechts im Maßstab 3 : 1 ein Detail vom Saugglockenrand in der Düsenumgebung.
Die Figur 11 zeigt oben einen Querschnitt durch eine Saugglocke mit einem anderen Mechanismus zur optischen Hautkontrolle mittels Fensters am Saugglockenrand. Unten im Maßstab 5 : 1 die Fensterumgebung.
Die Figur 12 zeigt im Längsschnitt in schematisierter Zusammenstellung in Aufsicht drei Gleichstrommagnete zur Funktionsauslösung in etwa natürlicher Größe.
Die Figur 13 zeigt in natürlicher Größe einen Längsschnitt durch das Detail eines Einkammerinjektors mit zwei Injektionskolben mit einem Druckgeber, der durch Druckgas gespeist wird(oben). In der Mitte das zugehörige Steuergetriebe für die Bewegung des hinteren Kolbens in Anlehnung an Fig.24. Unten im Maßstab 2 : 1 ein Detail in der Umgebung des hinteren Kolbens.
Die Figur 14 zeigt oben im Längsschnitt einen Einkammerinjektionszylinder mit einer speziellen Ventilkammer für dessen Füllung. Darunter ein Querschnitt durch die Ventilkammer.
Die Figur 15 zeigt links eine Gas-Druckspeicher-Kapsel vor einem Einkammerinjektionszylinder(rechts) im Längsschnitt in natürlicher Größe.
Die Figur 16 bringt in einem Längsschnitt in fast natürlicher Größe ein Versorgungsgerät mit Austausch-Behälter-Einheiten für die Versorgung eines Injektors mit Verdünner und Wasser während der Ladung der (nicht dargestellten) elektrischen Batterie. REchts wird im Maßstab 2 : 1 ein Längsschnitt durch eineVariante einer Behältereinheit innerhalb des Verpackungszylinders.
Die Figur 17 zeigt im Längsschnitt im Maßstab 2 : 1 das Detail einer Injektoröffnung, in welchen der Abfüllstutzen eines Versorgungsgerätes eingeschoben wurden.
Figur 18 ist ein Längsschnitt im Maßstab 3 : 1 durch eine Behältereinheit, bei der die Behälter übereinander angeordnet sind.
Die Figur 19 zeigt in natürlicher Größe ein Versorgungsgerät, auf das ein Injektor (links im Detail) aufgeschoben wird. Es ist der Schiebedeckel über dem Schacht für die Behältereinheit abgebildet, der während der Injektorverschiebung geöffnet wird. Unten ein schematischer Querschnitt die Schienenführung zwischen Injektor und Versorgungsgerät.
Die Figur 20 zeigt links im Längsschnitt eine Behältereinheit nach Fig.18 im Injektorschacht, darunter die Teleskopschraube des Versorgungsgerätes zur Einführung der Behälerteinheit. Der Maßstab ist 1 : 1.
Die Figur 21 zeigt im Längsschnitt eine Kette von Behältereinheiten innerhalb des Versorgungsgerätes und darüber einen Injektorschacht, wobei Druckluft zum Wechsel der Behältereinheit eingesetzt wird.
Die Figur 22 zeigt im Längsschnitt im Maßstab 1 : 1 ein Versorgungsgerät mit einer Nachfüllvorrichtung für je eine kleine Behältereinheit im Injektor aus größeren Verdünner- und Wasserbehältern. Oben links der Mitte ein Detail bei Klappendeckelöffnung nach Koppelung mit dem Injektor.
Rechts oben das Detail uni einen geschlossen Klappdeckel im Maßstab 3 : 1, darunter das Detail eines Vertikalschnittes durch den Gehäusezylinder zur Demonstration der Schlauchverbindung.
Die Figur 23 demonstriert in schematischem Querschnitt in natürlicher Größe oben die Einwirkung eines einzigen Druckgebers nacheinander auf zwei Injektionszylinder-Kolben. Darunter im Längsschnitt die Anordnung der Stahlfedern zu den Injektionszylindern und die Zuordnung letzterer zur Saugglocke.
Die Figur 24 zeigt oben in natürlicher Größe ein motorbewegtes Steuergetriebe mit Schiebspindel zum wahlweisen Antrieb von Rotations- und Schubfunktionen. Ein Schub-Torsions-Glied findet sich im Eingriff mit einem Schieber (wie in Fig.2), alles im Längsschnitt. Links unten im Vertikalschnitt die Zahnräder und Schaltelemente.
Die Figur 25 zeigt im Längsschnitt im Maßstab 3 : 1 oben ein abgewandeltes Schaltgetriebe, wie es nach Fig.9 zur raschen Dosierung von Arzneien eingesetzt werden kann.
Unter dem Dosiergetriebe eine Profilleiste zu dessen Betätigung über eine Verschiebespindel. Rechts das Detail eines Kugelschnäppers zur getrennten Mitnahme je einer Hälfte der Profilleiste im Vertikalschnitt. Rechts unten im Maßstab von 1 : 4 die beiden Profilleisten in Aufsicht in der Ebene auseinandergeschoben, da sie sich sonst gegenseitig teilverdecken.

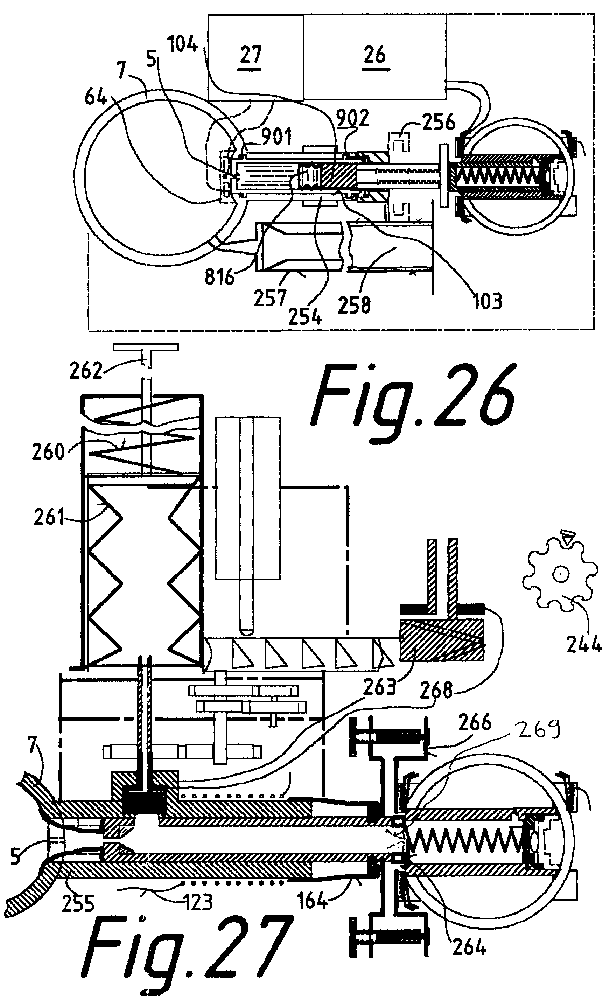

Die Figur 26 zeigt eine Injektion aus der Düse direkt in der Arzneipatrone im Längsschnitt und Maßstab 1 : 1.
Die Figur 27 wählt Maßstab 2 : 1 für einen Pulverinjektor.

### Ausführliche Beschreibung der Ausführungsbeispiele:

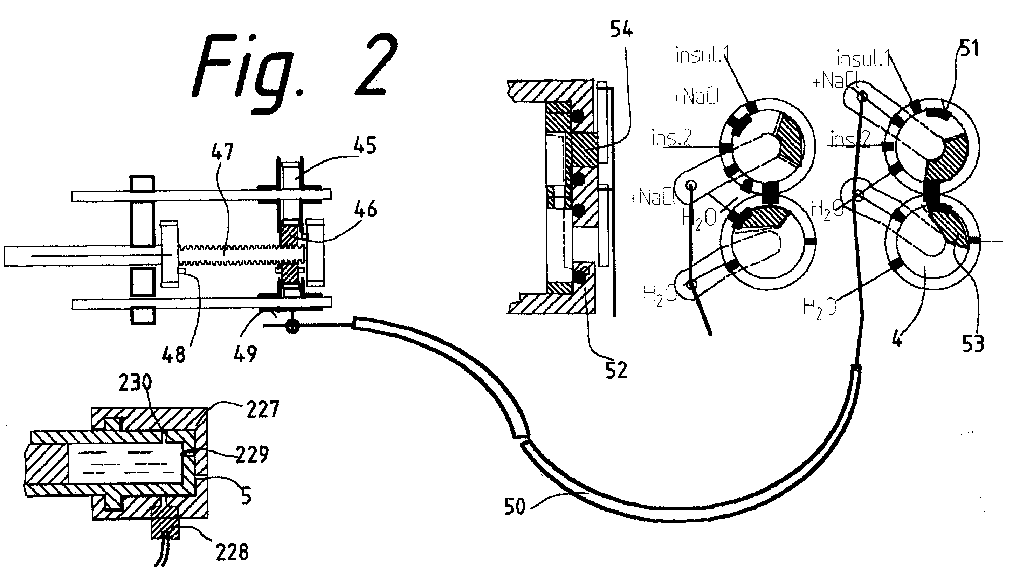

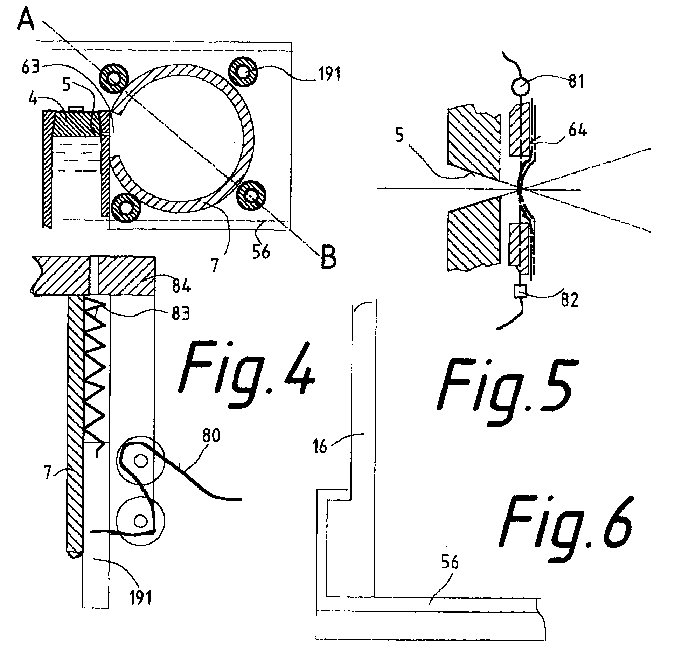

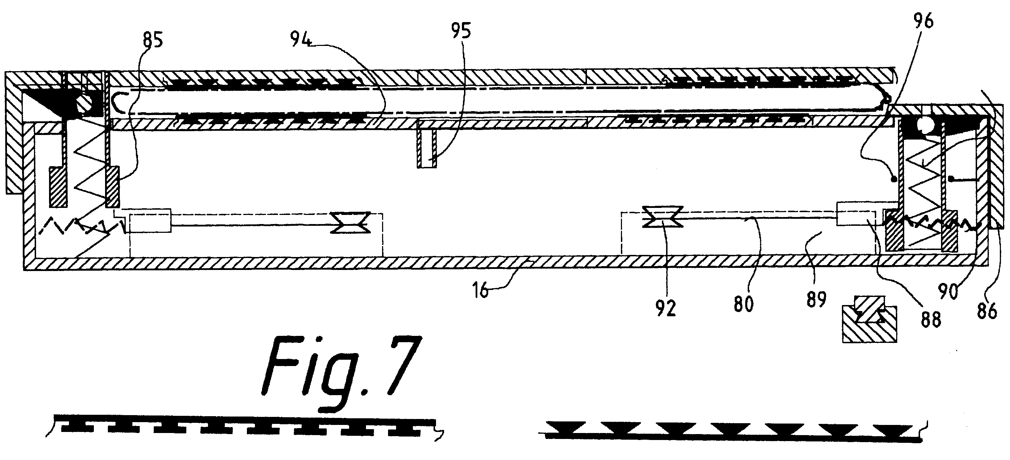

Die Figur 1 zeigt in einem schematisierten längsschnitt in natürlicher Größe die bevorzugte Lösung des Injektors, wobei die Gehäusewandung nur bruchstückweise gezeigt wird.
In der Mitte unten ist noch ein Detail des Mechanismus für die Injektionsverzögerung für den Injektionszylinder für Verdünner in Seitenansicht von unten dargestellt.
Die Schnittebene verläuft durch den unteren Injektionszylinder(1) für Verdünner und läßt die Keilschräge des Injektions(zylinder)kolbens(2) erkennen. Zwischen Injektionszylinder und -kolben spannt sich der Schutzfaltenbalg(3), welcher eine Hilfsflüssigkeit(etwa steriles Wasser) umschließt. In das sich leicht konisch verengende Zylinderende ist die Ventilscheibe(4) eingeschoben, welche die Düse(5) in Richtung Saugglocke(7) abdeckt (und freigeben kann). Die Ventilscheibe kann mittels des Hebels(6) gedreht werden(vgl.Fig.2). Die Sogauslösung erfolgt über die Anhebung der vier Auslösestifte(8), nachdem der Schiebedeckel(56) zurückgeschoben wurde. Die Auslösestifte sind neben der Saugglocke in Rohrsegmenten oder in erkerartiger Leiste gelagert(vgl.Fig.4 links unten). Die Rohrsegmente können auch bei Teilung des Beutels für die Sogerzeugung zwischen beiden Beuteln sich bis unter den Deckel(86) -an dessen Unterseite befestigt- erstrecken.
Sie werden dann nach Auslösung der Rasterschlitten(88) mit dem Deckel angehoben(vgl.Fig.7). Bei der hier gezeigten exzentrischen Lage der Saugglocke bleibt es bei einem entsprechend gekürzten Beutel(93,Fig.7). Das Fenster(9) mit der Lochblende(9) unterbricht die Saugglocke(vgl.Fig.5).
Die Wand des Injektionszylinders kann in der Düsenumgebung der Saugglocke als dichtender Abschluß dienen.
Der Kantstift(10) ist zwischen Kugeln gelagert und weist am Ende zur Abschrägung des Injektionskolbens die Rolle(11) auf. Am Handrad(12) kann der Schraubbolzen(13) höhenverstellt werden. Der Rasterschalter(15) meldet die Sektordrehungen gemäß Absenkung der Kugel durch ein rinnenförmiges Zickzackprofil an der Unterseite des Handrades gegen eine Feder durch Kontaktschluß an die elektronische Steinerzentrale(27). (Verdrahtungen und Schlauchverbindungen wurden in der Zeichnung weggelassen). Der Schraubbolzen wird durch die Gabelplatter(14) an einer Drehung gehindert, da letztere in seitliche schlitzförmige Ausfräsungen der Injektionskolben eingreifen(Detail Mitte unten). Die Gehäusewand(16) wird nur ein Stück weit dargestellt, zumal die Baulemente besserer Übersichtlichkeit wegen etwa auseinandergezogen dargestellt sind. Jeder der Kantstifte ist über den Hebel(17) starr mit der Schaltstange(28) verbunden. Auch letztere ist kantig und zwischen Kugeln gelagert.
In eine Auskerbung des Auslösebolzens greift mit entsprechender Ausfräsung ein Auslösestift(19) querverlaufend ein.
Die Bogensehne(38) spannt sich vom Auslösebolzen in der Mitte mit den beiden Enden zu den Enden der Stahlfedern(37).
Gespannt wurde die Bogensehne mittels der Gewindespindel(23) über das Getriebe(24) vom Motor(25), der aus der Batterie (26) gespeist wird. In der letzten Verkürzungsphase der Gewindespindel in ihrer Hülse wird über die Auslösegabel(22) -im gestrichelten Teil an deren Ende befestigt- der Kipphebel(20) so (über nicht dargestellte Zugrolle mit Bewegungsumkehr- bewegt, daß er der Auslösestift aus der Rastkerbe drückt. Die Zugfeder(21) holt den Kipphebel zurück.
Der Auslösestift wird vom Keil(29) zurückgeschoben, der sich unter Einfluß der Zugfeder(30) längs einer Schiene auf der Schaltstange(28) bewegt, wenn letztere über den Schaltstift mit den Hebel(17) verschoben wird.
Der Keilschieber(33) wird in einer Schlitzführung längs eines Stiftes verschoben. Letzterer steht fest auf dem Gehäuseboden. Die Verschiebung erfolgt über Vermittlung der Feder(32) über der Schwenkhebel(34). Letzterer weist die Drehachse(35) auf und kann über -nicht dargestelltem Gestänge mit dem Ende der Gewindespindel gekoppelt werden.

Es kann auch das Ende der Feder(32) mit dem Schiebedeckel (56) gekoppelt werden, so daß nach Rückführung des Kantstiftes(10) von der Keilschräge der Injektionskolben der Keilschieber(33) an der kleinen Rolle(79) seitlich der Injektionskolben angreifen kann. Sobald die Ventilscheibe den Flüssigkeitszufluß zu einer Zuleitung in der Injektionskolben frei gibt, kann letzterer von dem Keilschieber von der Düse wegbewegt. Der Injektionszylinder wird entsprechend gefüllt. Verdünner oder Wasser werden dabei durch komprimierte Luft aus dem Beutel(93,Fig.7) zwischen den Faltenbälgen und dem Pappzylinder(39) unter Druck gesetzt.
Der Überdruck wird im dargestellten Beispiel über das Rückschlagventil(43) auch nach Absenkung des Gerätedeckels und während der Ausdehnungsphase des Beutels im Behältergehäuse(135) festgehalten. Obgleich für den gegebenen Funktionszusammenhang nicht erforderlich, wird der Kragenring des Pappzylinders von der getrennten Mantelhülse(136), welche vom Deckel mittels des Klappgriffes(40) bei Drehung im Bajonettverschluß gegen den Dichtungsring(137) gepreßt wird. Da in unserem Falle eine Trennung in zwei Gasdruckkompartiments nicht erforderlich ist, gabelt sich der gemeinsame Luft-Zufuhrschlauch(42).
Die Arzneipatrone(138) und deren Entleerung wird auf Fig.9 näher beschrieben.

Die Figur 2 gibt eine Seitenansicht eines Schaltgetriebe-Details (links) in Kabelverbindung mit den beiden Ventilscheiben der Injektionszylindern (rechts) in natürlicher Größe. Links der an die Steuerung angeschlossenen Injektionszylinder ist eine andere Ventilstellung dargestellt, in der Mitte im Maßstab 2 : 1 ein Vertikalschnitt durch die beiden Enden der Injektionszylindern mit den Ventilscheiben. Links unten das Detail einer um einen Injektionszylinder kreisenden Ventilkappe mit exzentrischer Düse, die mit einer Öffnung im Zylinderboden zur Deckung gebracht werden kann. Seitliche Schlauchzuleitungen dienen der Flüssigkeitszuleitung über Bohrungen in der Kappe, die mit solchen im Injektionszylinder durch Rotation der Ventilkappe in Deckung gebracht werden können.
(Es wird nur ein Schlauch und eine Zylinderbohrung gezeigt).
Die Bohrungen können nach Distanz vom Zylinderboden gestaffelt zugeordnet werden. Der Zylinderboden kann entfallen, wenn auch die Düse im Winkel zur Kolben-Zylinder-Achse liegt.
Die Kantachse des Schaltgetriebes mit dem darauf verschieblichen Zahnrad(45) wird vom Motorgetriebe in Drehung versetzt. Es steht über Scheiben auf einer Achsmuffe mit dem Ritzel(46) in Schiebeverbindung. Die Verschiebung des Ritzels erfolgt bei dessen Drehung durch das im Eingriff befindliche Zahnrad längs der Schraube(47). Sowohl die Endteller der letzteren dem Ritzel zu als auch das Ritzel beidseits weisen Seitennoppen(48) auf. Eine parallel geführte weitere Kantachse dient der Seitverschiebung des Schlittens(49) mit einer das Ritzel umgreifenden Gabel.
Am Schlitten ist die Drahtseele des Bowden-Kabels(50) befestigt. Dessen anderes Drahtende verbindet starr die Enden der Schwenkarme der beiden Ventilscheiben.
Oberer und unterer Injektionszylinder stehen über einen Wandschlitz in gegenseitiger Hohlraumverbindung. Die Randkerbe(51) der Ventilscheiben wird mittels der Schwenkarme gedreht und passiert radiäre Bohrungen in der Wand der Injektionszylinder. Der eine Zylinder -in unserem Beispiel der obere- zeigt nacheinander die Bohrungen für die (als Striche symbolisierten) Zuleitungen für Insulin 1, für Verdünner (Wasser + Kochsalz), Insulin 2 und Wasser.
Der untere Zylinder zeigt nur die Einleitungen für Verdünner und Wasser. Die Breite der Randkerbe ist so gewählt, daß sie zur Weiterleitung je einer Insulinsorte dienen kann, in einer Mittelposition zwischen Insulineinmündung und Verdünneröffnung aber auch die Mischung Insulin-Verdünner einfließen lassen kann. Die Randkerbe kann bei entsprechender Stellung des Schwenkarmes auch ausschließlich Verdünner oder Wasser in die Injektionszylinder fließen lassen. In der extremen oberen Schwenkarmposition (linke Abbildung) ist der Flüssigkeitszufluß gestoppt, während der Flüssigkeitsausstoß aus beiden Zylindern durch die Düse im unteren Zylinder über die Aushöhlungen (schraffiert) erfolgen kann. Die andere Ventilscheibenposition (links) entspricht dem Zustand während einer Verdünnerauffüllung der Zylinder.
Das vergrößerte Detail in der Mitte im Vertikalschnitt zeigt die Lage zweier Dichtringe(52) und den Durchgriff der Schwenkarme mittels Achse(54) zu den Ventilscheiben.
(Hier wurden die Hohlräume ohne Schraffur gelassen).
Während des Wasserausstoßes mit herabgesetztem Druck kann das Wasser gegebenenfalls auch in den Raum zwischen Ventilscheibe und Dichtring zur Reinigung eintreten.

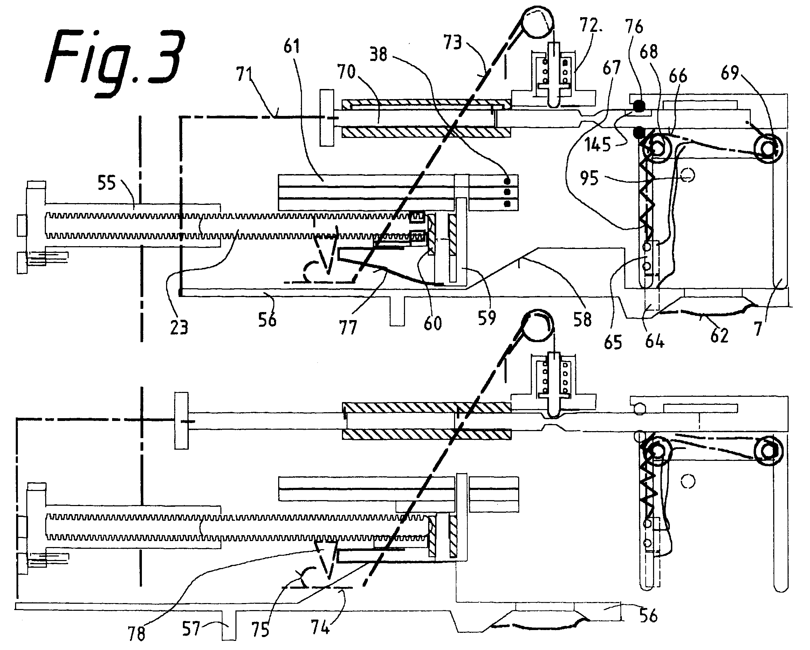

Die Figur 3 zeigt im Querschnitt längs der Schnittlinie A - B der Fig.1 weitere Funktionsmechanismen und deren mechanische Grundlagen in natürlicher Größe und zwei Funktionsstadien.
Die Gewindespindel(23) ist hier jeweils aus ihrer Gewindehülse(55) herausgedreht.
In dere oberen Darstellung ist der Schiebedeckel(56) nach rechts in seiner Schienenführung(vgl.Fig.6) herausgezogen, so daß er die Saugglocke(7) abschließt. Unter dem zentralen Durchlaß findet sich die Klammer(62) zur Befestigung eines saugenden Zellstofftupfers, welcher das Wasser während einer Reinigungsprozedur für Zylinder und Düse aufnehmen kann. Im Schlitz(63,Fig.4 oben) innerhalb der Saugglockenwandung ist ein Schieber vertikal beweglich, dessen unterer Teil dar Fenster(64) mit der Lochblende enthält, während der obere Teil eine hitzestabile Dichtplatte mit Glühdraht aufweist, welche in der dargestellten Position die Düse abdichtet. Der untere Teil des Schiebers überragt den Saugglockenrand unter Einfluß der Druckfeder(67).
Letzterer wirkt der Seilzug(66) entgegen, welcher über die Umlenk-Rollen(68,69) geführt und am Ende des Raststiftes (70) befestigt ist. Der mit dem Gerätegehäuse fest verbundene Auslöseraster(72) ist nach Betätigung des Seilzuges (73) ausgelöst. Die Spannung der Feder(67) über Rückzug des Raststiftes bis zu dessen Einrastung erfolgt über das Schaltgestänge(71) zwischen Ende des Raststiftes und des Deckelschiebers, wenn letzterer an seiner Querleiste(57) zurückgeschoben wird (wie unten abgebildet). Die Querleiste kann sich noch zweckmäßiger ganz links am Ende des Schiebedeckels befinden. Von den elektrische Leitungen zu den Teilen des Schiebers ist jeweils nur eine und nur streckenweise eingezeichnet. Der Raststift(70) ist zur Saugglocke durch den Dichtring abgeschlossen.
Durch eine Halterung vom Ende der Gewindespindel(23) aus wird der Mitnehmerkeil(78) gehalten der auf die Krümmung der Blattfeder(75) einwirkt, die ihrerseits längs der Schiene(74) verschieblich ist. Bei seiner Bewegung nach rechts überholt der Mitnehmerkeis die Blattfeder(75), während er sie bei der Rückkehr nach links mitnimmt und dabei über den Seilzug den Auslöseraster(72) betätigt.
Die Blattfeder(77) senkt den kantigen U-Haken(59) ab, der mit einem Ende in die Bohrung des unteren Schubladenschiebers(61) hineinragt und diesen festhält. In dieser Geräteversion ist jeder der drei Stahlfedern(37,Fig.1) mit eigener Bogensehne(38) mit je einem Schubladen-Schieber verbunden. Der U-Haken wiederum verläuft innerhalb des Hülsensegments(60), welches mit dem Ende der Gewindespindel drehbar verbunden ist. Wird -wie in der oberen Abbildung- die Gewindespindel in die Gewindehülse gedreht, so wird dabei nur die untere Bogensehne gespannt. Nach Verrasterung in gespanntem Zustand(vgl.Fig.1) kann die Entleerung von Wasser durch die Düse mit verminderter Kraft und Geschwindigkeit erfolgen.
Bei Rückzug des Schiebedeckels -wie in der unteren Abbildung- hat die Keilschräge(58) gegen die Blattfeder(77) den U-Haken angehoben, so daß jetzt alle drei Stahlfedern gespannt werden können.

Die Figur 4 zeigt oben in einem Horizontalschnitt in natürlicher Größe das Detail eines Injektionszylinders mit Ventilscheibe(4) und Düse(5). Letztere ist (nach Entfernung des Schiebers mit der Lochblende und Heizspirale) gegen die Saugglocke(7) gerichtet, die unten durch den Schiebedeckel(56) abgeschlossen wird. Außerdem sind die vier Auslösestifte(8) in ihren Hülsen erkennbar.
Unter dem Hosrinzontalschnitt wird im Querschnitt im Maßlängs der Schnittlinie A - B des Horizontalschnittes im Maßstab 2 : 1 ein Teil der Saugglocke(7) und ein Auslösestift(8) gezeigt, der innerhalb einer Bohrung des Erkervorsprungs (der hier die Hülse ersetzt) von der Druckfeder(83) niedergehalten wird. Der Seilzug(80) ist am Auslösestift befestigt und führt über zwei Rollen in einem Schlitz des Erkervorsprungs zu einem der vier Rasterschlitten(88,Fig.7) für die Sogauslösung.

Die Figur 5 zeigt in einem Längsschnitt durch den Schieber mit dem Fenster(64) und die Düse(5) in der Injektionszylinderwand im Maßstab 4 : 1 (wobei die Düse noch weiter größenmäßig überzeichnet wurde). Die strichpunktierte Linie zeigt die Haut, die sich dem Fensteranlegt und in die Lochblende hinein vorwölbt. Der von der Lichtquelle (81) zum Lichtempfänger geführte Strahl verläuft in einer Fensterbohrung (oder in Lichtleitfasern mit Linsen zur Öffnung in die Lochblende hin) und streift dabei gerade die kleine Hautblase. (Vom Lichtempfänger führt das Kabel zum Photometer innerhalb der elektronischen Steinerzentrale(27,Fig.1). Der Flüssigkeitstrahl (gestrichelt) wird durch die konische Düsenverengung zwischen der Lochblende gebündelt.

Die Figur 6 zeigt im Vertikalschnitt im Maßstab 2 : 1 die Befestigung des Schiebedeckels(56) an einer Gehäusewand(16) als Detail.

Die Figur 7 gibt in etwa natürlicher Größe einen Vertikal-oder Querschnitt durch das Erfindungsbeispiel der Fig.1; die Schnittebene liegt in der Tiefe der Rohrstutzen (85), welche am Ende einen Randring aufweisen. Die Rohrstutzen sind am Deckel(86) über eine gummielastische Plattenverbindung(779) befestigt. Dies erlaubt einen auch etwas ungleichen Deckelschluß unter leichter Verkantung.
(Wozu allerdings zwischen Geräterand(16) und Deckelrand dann Freiraum, d.h.Spiel gelassen werden muß). Eine von der der Gerätewand ausgehende Käscher-Halterung gewisser Elastizität oder eine Art (nicht dargestellter) Hilfstrichter am Geräteboden sichert dabei, daß der Randring des Rohrstutzens am Rasterschlitten(288) vorbeikann. Dies ist allerdings erst möglich, wenn der Rasterschlitten auf seiner erhobenen Schiene(89) mittels des Seilzuges(80) über die Rolle(92) vom Auslösestift her zurückgezogen wird.
Zuvor bleibt die geöffnete Stellung des Deckels gesichert.
Links sind Deckel und Rohrstutzen in angehobenen, rechts in abgesenktem Zustand eingezeichnet. In einem Detail unten rechts wird die Schwalbenschwanz-Führung des Rasterschlittens in seiner Schiene sichtbar. Die Rückstellung des Rasterschlittens erfolgt über die Zugfeder(90). Die vier Seilzüge müssen über die Auslösestifte zugleich betätigt werden, um die Deckelanhebung freizugeben. Der Beutel(93) zwischen Deckel(86) und Deckplatte(94) des Gehäuses ist über den Verbindungsschlauch(95) mit der Saugglocke(7, Fig.3) verbunden. Paarweise zusammengestellte Teilsegmente der Beuteloberfläche zeigen Beispiele (links als Schwalbenschwanz, rechts als Mäander) für eine feste Verbindung zwischen Beuteloberfläche und Deckelunterfläche einerseits und Deckplatte andererseits. Die Lage der von innerhalb der Rohrstutzen auf den Gehäuseboden herabragenden Druckfedern -etwa nahe den Gehäuseecken- wurde in Fig.1 nicht verzeichnet.

Die Figur 8 zeigt die Lösung für den Antrieb eines Teiles des mechanischen Steuergetriebes durch einen kleinen Elektromotor(97). Von der Motorachse des kleinen Elektromotors verläuft der Kraftfluß über ein kleines Zwischengetriebe(98) zur Minderung der Drehzahl und setzt sich dann über die Übersetzungszahnräder(99) zur Arbeitsachse fort. Diese kann eine Dosierspindel betätigen, wobei dann zweckmäßigerweise zwei solcher Vorrichtungen für die Dosierung eingesetzt werden. Es kann so rascher eingespritzt werden.

Die Figur 9 zeigt zwei zu Zwecken eines Dosierspindelantriebes zusammengeschaltete Hubmagnete(100,101) im Maßstab 2 : 1. Die Magnetanker wirken mit ihrem abgerundeten Ende gegen kleine Keilzähne eines (in Aufrollung und teilweise gezeigten) Zahnrades. Vom linken Magneten(100) wird über ein Sperrzahnrad das Zahnrad(102) in kleinen Teilschritten gedreht, welches die Dosierspindel antreibt. Die Arbeit des Magneten(101) erfolgt entsprechend, jedoch wird die Drehwirkung auf das Zahnrad (102) über das Zwischengetriebe(98) erhöht. Die gegenläufig angeordneten Sperrzahnräder bewirken zusammen die Drehung dem Zahnrades(102) oder behindern einander wenigstens nicht. Grob- und Feindosierung und damit Gesamtdosierung können so rasch für eine Arzneisorte erfolgen.
Die Blattfeder(143) stabilisiert gegen die wellige Oberberfläche des mit dem Zwischenrad(108) verbundenen Rades (144, Detail vertikal geschnitten rechts) dessen Sektorbewegung und zählt die Dosen durch Kontaktbetätigung in Leitungsverbindung mit der Steuerzentrale.
Das Zahnrad(107) auf der Mikrometerschraube(106) wird durch die Gabel(134) in Eingriff mit dem Zwischenrad(108) gehalten und am seitlichen Ausweichen gehindert. Bei Drehung des Zahnrades(102) mittels der Magnete(100,101) wird die Mikrometerschraube nach links bewegt und treibt den Stopfen(140) innerhalb der Arzneipatrone(138) vor sich her. Die Arznei wird dabei über die abgewinkelte Kanüle(105) in den Injektionszylinder(1) verdrängt.
Während Fig.8 und Fig.9 hauptsächlich im Horizontalschnitt (bei Aufrollung der Keilprofilräder in Fig.9) dargestellt wurden, zeigt der Vertikalschnitt links oben die Lage der Zwischenzahnräder(109,110), die über Linksverschiebung des Gestänges(111) unter Abdrängung des Zahnrades(108) in Eingriff mit dem Zahnrad(107) eine Bewegungsumkehr der Mikrometer über Magnetaktionen bewirken. Erst nach Rückführung der Mikrometerschraube kann die Arzneipaatrone ausgetauscht werden. Die Kappe in Verbindung mit der Kanüle wird durch den Schraubstock(141) festgehalten, die Arzneipatrone durch die Klammer(142).
Die Faltenbälge für Verdünner und Wasser innerhalb des Pappzylinders(39,Fig.1) können dergestalt abgewandelt werden, daß sie -einen maximalen Tagesvorrat fassend- vom Ladegerät für die Batterie her automatisch ausgewechselt werden. Es kann diese Behältereinheit aber auch im Injektor verbleiben und täglich aus größeren Vorratsspendern nachgefüllt werden.
Vor der Injektion werden auf dem nicht dargestellten Tastenfeld auf der Geräteoberfläche die Kohlenhydratmengen, deren Einnahme beabsichtigt ist (etwa als Dreiecke mit der Spitze nach unten gemäß dem Glukoseabfall unter Insulineinwirkung) und die geplanten körperlichen Belastungen (etwa als Rechtecke verschiedener Breite) einer wandernden Zeitleiste zugeordnet und weitere Programmierungen an Drehknöpfen an einer Geräteschmalseite vorgenommen.
Die Unterscheidung zwischen größeren Injektionsabständen und kleineren -wie bei der intensivierten Insulintherapekann am Handrad(12,Fig.1) ins Programm übersetzt werden.
Durch Schalterbestätigung -etwa nach Erinnerung durch eine Warnvorrichtung- steuert der Motor die Druckgeberspannung und den Zufluß der vorgesehenen Insulinsorten und des Verdünners in die Injektionszylinder. Es wird der Schiebedeckel von der Saugglocke zurückgeschoben und letztere gegen die Haut gepreßt. Nach Sogauslösung(Fig.4,7) erfolgt -etwa über eine Kontaktöffnung zwischen Deckel(86) und Deckplatte(94,Fig.7) die Auslösung der optischen Hautkontrolle (Fig.3,5) und von der Steuerzentrale aus über die Schraube (47,Fig.2) die Auslösung der Druckgeber und damit der Injektion. (Bei Nichteignung der Punktionsstelle wird über vorzeitige Bewegungsumkehr der Gewindespindel(23,Fig.3) an der Blattfeder(75) die Wiederbelüftung eingeleitet; wie sie auch über die Rinne(145) unter dem Dichtring(76) erfolgen kann). Zur Wiederbelüftung drängt der Schieber mit dem Fenster(64) die Haut vom Saugglockenrand ab.
Es kann ein Zellstofftupfer unter die Klammer(62) geschoben und der Reinigungszyklus von der Steuerzentrale durch Drehung eines (nicht gezeigten) Schalters oder automatisch abgerufen werden. Auch die Schließung des Schiebedeckels(56,Fig.1,3) kann automatisiert werden. Über eine (Blatt-)Feder etwa am Schwenkhebel für die Ventilscheibe (4,Fig.2), welche über eine Steilflanke geführt wird (ähnlich dem Mechanismus der segmentalen Rasterung der Drehung des Rades(144) in Fig.9) kann die Öffnung der Düse mit der Druckgeberauslösung synchronisiert werden.
Zwischen Dichtplatte(65,Fig.3) und Düse bildet sich eine Kammer mit Unterdruck durch Dampfsterilisation, wie sie nach Wasserdurchspülung mittels Glühdrahtschlinge durchgeführt wird. Aus andere hygienisch kritische Stellen -wie etwa die Einfüllstutzen für Wasser urd Verdünnerwerden zweckmäßigerweise durch Heizdrahtschlingen gesichert. Die Deckelabsenkung unter Auffüllung des Druckgasdepots im Behälter für die Faltenbälge mit Verdünner und Wasser kann zu beliebigem Zeitpunkt vor einer beabsichtigten Injektion erfolgen. Das Überdruckventil(44, Fig.7) gewährleistet dabei etwa gleichartigen Kraftaufwand.

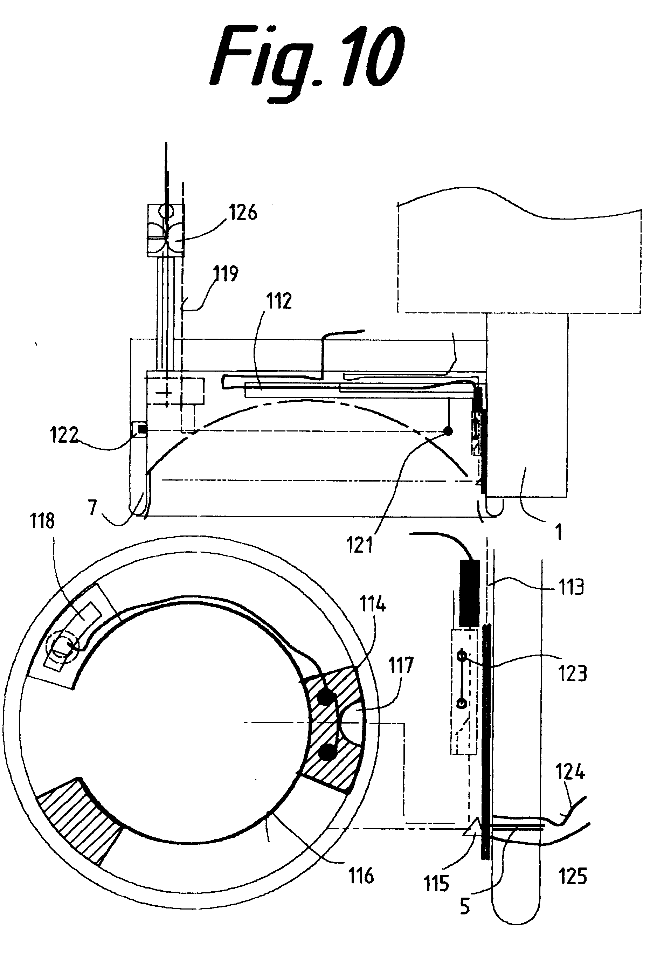

Die Figur 10 gibt eine alternative Lösung für die Düsenabdeckung außerhalb der Injektion von Arznei oder Ejektion von Wasser zur Reinigung. Diese Abdeckungen werden hierdurch vor der Druckgeberaktivierung aus dem Düsenbereich weggedreht bzw.angehoben.
Oben ein Vertikalschnitt durch die Saugglocke im Maßstab 2 : 1 . Darunter links ein Horizontalschnitt im Maßstab 1 : 1 in Höhe A - B (also in Strahlhöhe) des Vertikalschnittes als Detail der Umgebung des Injektionszylinders.
Rechts unten wird im Maßstab von etwa 3 : 1 die Düsenumgebung näher illustriert.
Der Querbalken(112) wird längs der Rillenschiene(113) durch die unter Sog sich hebende Haut mit hochgehoben.
Angehoben wurden dabei auch die sektorartige Mantelhülse (114) mit ihrer Heizdrahtschlinge samt Zuleitungen und Photoemitter und Photosensor mit Leitungen vom Saugglockendach aus. (Jeweils wurde nur ein Leitungsende dargestellt im Detail rechts unten, und der Leitungsverlauf innerhalb der Saugglocke im Längsschnitt oben).
Der die Hautbeschaffenheit kontrollierende Lichstrahl fällt beispielsweise vom Photoemitter senkrecht auf die dreieckige Spiegelnase(115) auf der Innenseite des Sektors der Deckelblende(116). Für den Vorbeizug der Spiegelnase und den Lichtdurchlaß weist die Mantelhülse(114) den Durchlaß(117) auf. Der Keil(120) an der Auslösestange(119) bewirkt bei Absenkung (oder in anderen Fällen bei Anhebung) eine Verschiebung der Lasche der Deckelblende am Führungsschlitz(118) und damit die Freigabe der (übertrieben groß gezeichneten) Düse(5) und des Lichtweges zur Haut durch Radiärbewegung mittels des elastischen Bügels(151) schon vor Anhebung des Querbalkens(112) durch die sich hebende Haut. Die Ausstattung zur Wiederbelüftung entspricht derjenigen in Fig.13.
Der Photoemitter(121) und Photosensor(122) ist mit seiner Laserstrahlenverbindung für die verletzungsfreie Stoffwechselmessung -insbesondere für Glukose- ohne Leitungsverbindungen und zugehörige analytische Apparatur eingezeichnet. Entsprechende lichtoptische Verfahren wurden in der Bundesrepublik zuerst von Nils Kaiser (Untergarching) und Arnold Müller(Ulm) zum Patent angemeldet.
Als Alternative für den Heizdraht(123) in der Blende(unten) wurde oben der Heizdraht um die Düse gelegt. (Letztere am strich-punktierten Flüssigkeitsstrahl zu erkennen). Als Alternative zur optischen Überwachung finden sich unten in der Nähe der Düse Lichtleitfasern(124,125) zum Emitter und zum Sensor eingezeichnet, so daß Licht von der Haut reflektiert und ausgewertet werden kann.

Die Figur 11 zeigt einen optischen Hautkontrollmechanismus innerhalb einer Saugglocke (oben in natürlicher Grösse) mit (eventuell) steuerbarem Drosselventil(126).
Die Darstellung bedient eines Vertikalschnitts.
Der Injektionszylinder(1) ragt unterhalb des Druckgebers (127, gestrichelt, weil hinten liegend) in die hinten angeschnittene Saugglocke(7). Am Saugglockenrand liegt das transparente Glas- oder Kunststoff-Fenster(128) unmittelbar unter der Düse(5). Daneben -beispielsweise noch im Saugglockenrandbereich- die Photomeßausstattung mit Photoemitter(121) und Photosensor(122).
Unten ist im Maßstab 5 : 1 das Detail der optischen Kontrolleinrichtung wiederholt. Gitterartig ist die Hautfelderung unter dem Fenster(128) eingezeichnet, dazu der Lichtstrahlenverlauf vom Phototransmitter (etwa einem LCD oder Laser) durch das Fenster, dort von einer spiegelnden Konvavkante gegen die Haut zurückgeworfen und von dieser durch das Fenster zurück über dessen Konvexkante in den Photosensor. Von den Kabelverbindungen sind jeweils nur zwei an ihren Endabschnitten wiedergegeben.
Während der Hautanhebung wird das Hautmuster am Fenster vorbeigezogen. Aus der Geschwindigkeit der Hautbewegung ordnet der Rechner nach dem Stillstand dieser Bewegung aus den gespeicherten Hautteilfeldmeßfeldern dem über der Düse bzw. über der Blende am Ort der Düse liegenden Meßfeld die entsprechenden Lichtexstinktionen zu.
(Eine Aufgabe, welche von jedem PC-CAD-Programm heute analog geleistet wird). Es kann so noch vor der Öffnung der Deckelblende über einen Abbruch des Funktionsablaufes ohne Injektion entschieden werden. Kommt bei einem gesprengelten Oberflächenmuster der Haut (etwa bei Akne oder bei Sommersprossen) eine pigmentiertere oder sonst wie von normaler Vergleichshaut in ihren optischen Eigenschaften abweichende Hautstelle in eine Lage über der Düse, so kann über Öffnung des eng gestellten Drosselventiles(126) die Hautglocke in der Saugglocke leicht abgesenkt und die Injektion in einem Augenblick vollzogen werden, in welchem eine wahrscheinlich gesunde oder doch weniger empfindliche Hautstelle über der Düse liegt.

Die Figur 12 zeigt im Längsschnitt in schematischer Zusammenstellung drei Gleichstrommagnete zur Funktionsauslösung und ihre Funktionsorgane in etwa natürlicher Größe. Die gestrichelte Verlängerung des Ankers(129) des Druckmagneten(130) bezeichnet den Arbeitsweg(557). Es wird der Schalthebel mit dem Keil(334) beaufschlagt, der im Detail oben über dem zugehörigen Führungsschlitz der Lasche der den Injektionszylinder(67) umgebenden Mantelhülse(114) in Segmente gespalten federnd anliegt. Das Ende des Injektionszylinders weist eine nach oben abfallende Konizität auf. So wird die die Mantelhülse abschließende Deckelblende gegen die in Fortsetzung der Injektionszylinderachse angebrachte Düse(5) gepreßt.
Der Vertikalschnitt links in Richtung der Schnittlinie A - B läßt eine Sektoröffnung(182) zur Freigabe der exzentrischen Düse und Anschlag(133) und die gegen letzteren wirkende Rückstellfeder(134) an der von der Injektionszylinderwand rechtwinkelig abstehenden Lasche der Mantelhülse erkennen. Durch Drehung des Schalthebels mit Keil(120) um 90 Grad wird dessen Form im unteren Detail in Verlängerung des Magnetankers (also im Querschnitt) erkennbar. Der Zugmagnet(132) wirkt über den Seilzug(80) und die Rolle(92,vgl.Fig.7) auf den Rasterschlitten(88) gegen dessen Zugfeder(90). Durch Freigabe des Rohrstutzens(85) können die vier Druckfedern(181) die Ausdehnung des Beutels(93,Fig.7) bewirken. Es tritt nun Unterdruck in der Saugglocke auf.
Der Zugmagnet(132) wirkt über seinen Anker und den Seilzug(73,Fig.3) über eine Rollenführung auf den Bolzen des Auslöserasters(72) ein. Dadurch wird der Raststift(70) frei und die Wiederbelüftung der Saugglocke bewirkt.
Der Druckgeber -z.B.Kantstift(10,Fig.1) über Zug am Auslösestift(19)- für die Ejektions- oder Injektionauslösung kann über einen weiteren Zugmagneten erfolgen.
Anstelle der Variante mehrerer Elektromagnete kann auch ein Multifunktionsmagnet eingesetzt werden, dessen Anker auf eine Schub-Torsions-Einheit(180) unter sektoraler Achsendrehung verschiedene Funktionsabnehmer in radiärer Anrordnung nacheinander bedient.(Detail rechts oben im Horizontalschnitt).
Die verschiedenen Auslösefunktionen können auch vom Motor aus betätigt werden, etwa durch Rotationsabgriff durch Arbeits-Zahnräder, während deren Eingriff mit dem Ritzel (46,Fig.2) längs der Schraube(47).

Die Figur 13 zeigt in einem Horizontalschnitt durch das Detail eines Injektionszylinders nach dem Zweikammersystem eine Alternative zur Lösung des Problems der Injektionskanalauswaschung durch Verdünnungsflüssigkeit im Maßstab 2 : 1.

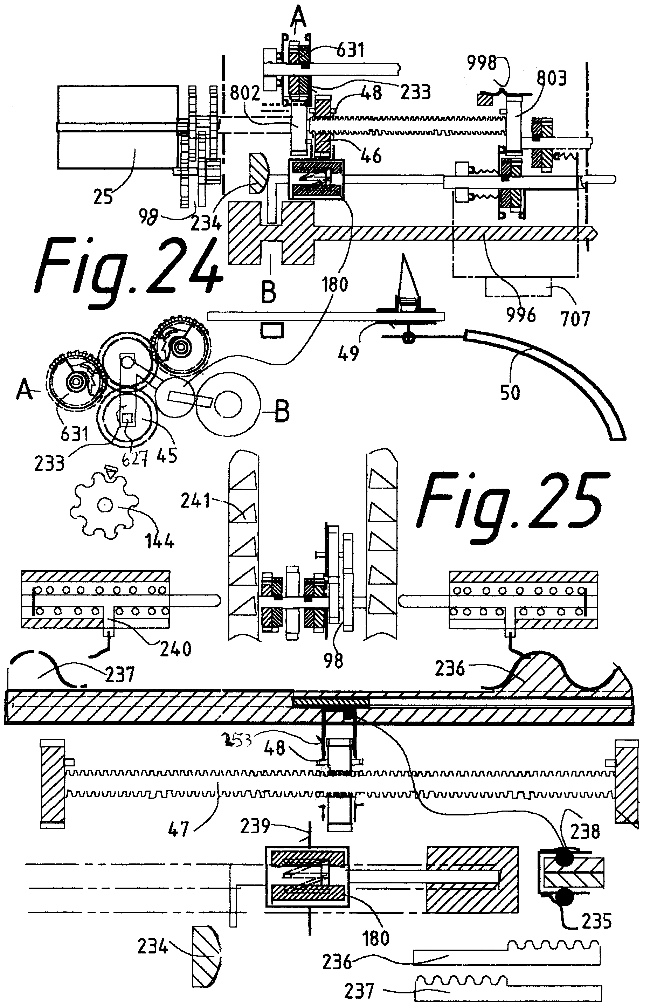

Als Druckgeber für die Doppelkolbenanordnung im Injektionszylinder dient ein ovaläres oder eckiges Gehäuse(146) mit der Zwischenwand(147) für die Anlage des Faltenbalges (148). Die GEwindehülse(149) wirkt drehend auf das Gewinde des Hammerbolzens(437), dessen Hülse in der nicht ganz durchgehenden Binnenbohrung(152) des hinteren Kolbens mit einer tellerartigen Verbreiterung freidrehend endet.
Die Gewindehülse wird mittels des Zahnrades(153) auf der Zwischenwand(147) von einem darüber liegendenden in Platterverbindung bei Absenkung mitgenommen (nicht dargestellten) Ritzel angetrieben. Letzteres steht über die biegsame Welle(154) mit der Welle des (hier funktionslosen) Zahnrades(155) und über die Welle mit dem in der Mitte (also darunter) abgebildeten Steuergetriebe in Verbindung. (Das Steuergetriebe entspricht demjenigen der Fig.24. Der Bewegungsableitung zur Antriebswelle erfolgt hier aber nicht über ein Sperrzahnrad, sondern wirkt in beiden Richtungen verschiebend auf den hinteren Kolben (165) zur Regelung des Verdünnerzuflusses über den Verbindungsschlauch(170) und die Bohrung(231) mit dem Klappenventil (siehe auch Detail unten). Die Füllung des Injektionszylinders zwischen Düse und Injektionskolben(2) erfolgt über eine der Zuleitungen über das Klappensegel (176) unter Kolbenverdrängung. Der hintere Kolben(165) ist durch eine Lasche(232) zum Gehäuse gegen Drehung gesichert. Vor der Injektion wird der Staudruck im Faltenbalg(148) wird über (links oben nur als Kreise angedeutete) Kanalverbindungen aus einer (nicht dargestellten) Druckgaskapsel über den Druckstaubehälter(158) eingeleitet. Der Riegel oder Innenschieber(159) ist gegen den Kantenvorsprung(160) angeschrägt und steht unter der Einwirkung einer starken Druckfeder, die schließlich vom Gasstaudruck überwunden wird. Dabei wird der kleine Riegel am in einem Schlitz vom Schachtelboden(163) mitgenommenen Bowdenkabel(162)betätigt und damit eine Blende über der Düse(5) innerhalb der Saugglocke(7) weggeschoben(vgl.114,Fig.12). Zwischen Hammerhülse(171) und Injektionszylinder(1) ist die elastische Schutzmembran(164) ausgespannt. Düsenwärts unten blind endende Längsrillen(166) im Injektionskolben(2) kommen im Endvorschub bei der Ausspritzung mit entsprechenden Längs-(168) und Boden(169)-Rillen des Injektionszylinders in Deckung, so daß der zwischen vorderem und hinterem Kolben gestaute Verdünner ausgespritzt werden kann. (Entsprechendes gilt für den Reinigungszyklus mit Wasser). Die in die Saugglocke(4) gerichtete Düse(5) sind eingezeichnet, ebenso die Zwischenwand(147) als Gegenlager für den Faltenbalg.

Die Figur 14 gibt oben einen Horizontalschnitt durch den vorderen Abschnitt eines Injektionszylinders(1) nach dem Einkammerprinzip wieder. Unten wird im Querschnitt die Ventilkammer(172) demonstriert. Der Maßstab ist 3 : 1.
Es gibt bei der hier dargestellten Lösung nur einen einzigen Injektionskolben(2) als Druckgeber. Die Düse(5) ist beispielsweise frontal angeordnet, die Schutzklappe(173) (von elastischer Beschaffenheit) wird zur Injektion von der Düse abgeschwenkt. Die Zylinderbohrung wird schmal gewählt, und die Ejektion der Flüssigkeit erfolgt in Pfeilrichtung mit hoher Geschwindigkeit. Die Zylinderfüllung geht unmittelbar voraus. Dabei wird unter Rückzug des Kolbens mit hohem Druck Verdünner aus dem Kanal(174) gegen das elastische Septum(161) gepreßt, das sich auch der gegenüberliegenden Öffnung des Kanales(175) für die Wasserzufuhr eng anlegt. Durch den Druck bahnt sich der Verdünner seinen Weg in die Ventilkammer und verstärkt die Abdichtung des Kanales(175); auch schließen sich die Klappensegel(176,177) zu den Insulinpatronen und öffnet sich das Klappensegel(190) in den Injektionszylinder.
In einer zweiten Kammerfüllungsphase, wird Insulin dosiert von den Dosiervorrichtungen(vgl.Fig.8,9) durch die Klappensegel(176,177) in die Ventilkammer ausgestoßen und vom Verdünnerstrom in den Injektionszylinder getrieben. Die sofort anschließende Ejektion drückt das Klappensegel(190) gegen ihren Sitz in der Zylinderwand.

Der Reinigungszyklus erfolgt entsprechend mit Wasser.

Die Figur 15 zeigt in einem Horizontal schnitt einen sphärischen Austausch-Druckgasbehälter(178) -etwa für CO₂- mit Innenstutzen(179) für den Druckstößel(183).
Die Abdichtung vor Gebrauch erfolgt durch den Preßsitz des Druckstößels mit dessen Konizität(189) am Ende und an der Ringkante(184) mittels eingelassener Glühdrahtschlinge oder einer begrenzten leitender Materialzone, die gegen die Umgebung elektrisch isoliert ist. Die zentrale Ausrichtung gegen den Injektionskolben(2) erfolgt beispielsweise durch den Randschieber(135) an der Stutzenkante, die in einer auf dem Injektorboden feststehenden Leitgabel(186) geführt wird, ergänzt durch die Gegenlamelle oder -flosse(187) in einer Geräteschiene. Die Höhenjustierung kann durch den geöffenten Schiebedeckel(56, Fig.1) erfolgen. Abschmelzung der Ringkante durch Gleichstrom gibt die Bewegung des gegen den Injektionskolben gerichteten Druckstößels(183) frei. Eine derartige Ausstattung kommt der Miniaturisierung des Injektors zugute.
Von der Zweikolbenspritze ist der Injektionskolben(2) und der Verbindungsschlauch(170) zur Füllung des Zwischenkolbenraumes bezeichnet.
Das Detail unten rechts zeigt eine Variante von größerer Funktionssicherheit. Der Druckstößel ist durch eine Kartusche (oder einen Hohlstößel,196) ersetzt. In ihm steht die starke Druckfeder(198) gegen den Deckel(197) mit Randtülle gegen den Gasdruck in Spannung. Letzterer wirkt sich über die Gaszufuhröffnung(201) im Innenstutzen(179) aus, der den Deckel gegen den Dichtungsring(200) preßt.
Die Druckfeder stößt den Deckel in die gestrichelt dargestellte Öffnungsposition, sobald nach Abschmelzung der Dichtungsplombe (etwa aus Wachs) in der schmalen Einlaßöffung(199) im Innenstutzen durch Aufheizen desselben mittels des Heizdrahtes(123) der Gaseintritt ermöglicht wird. Gleichzeitig wird durch weitere Heizdrähte die Ringkante(185) um die Öffnung des Innenstutzens für den Hohlstößel abgeschmolzen, so daß letzterer aus dem Austausch-Druckgas-Behälter(178) herausgeschossen wird.

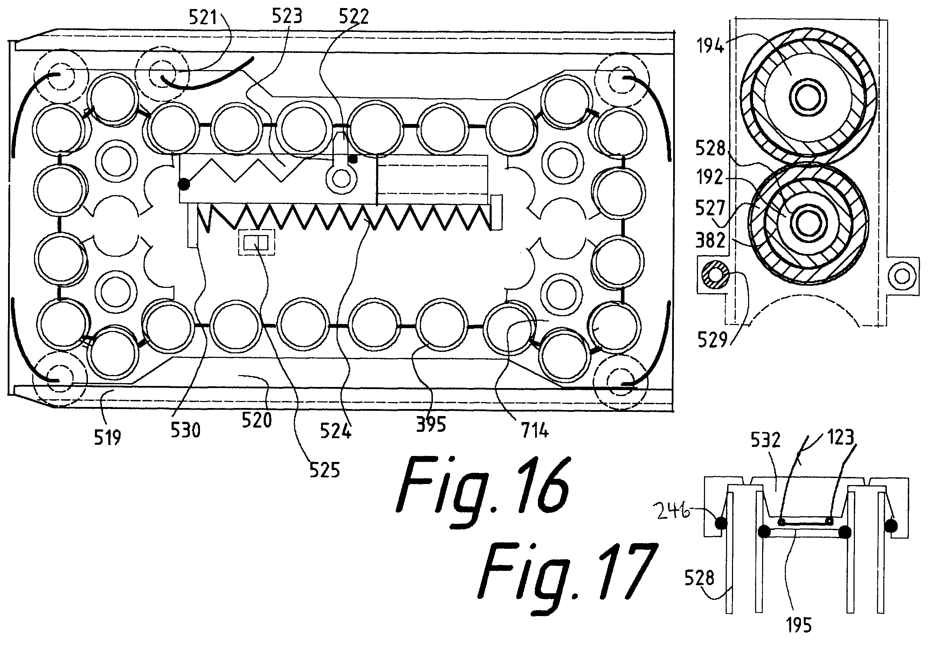

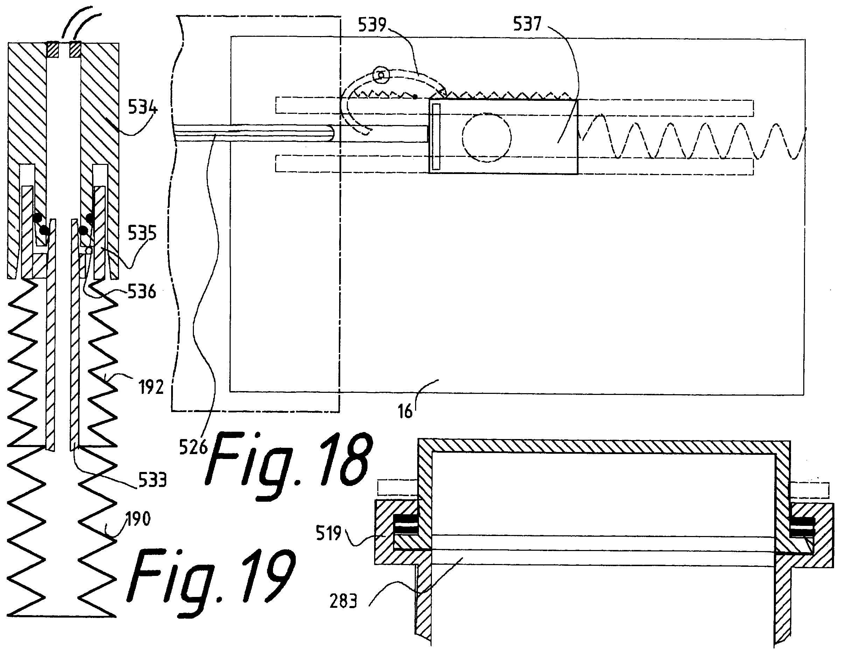

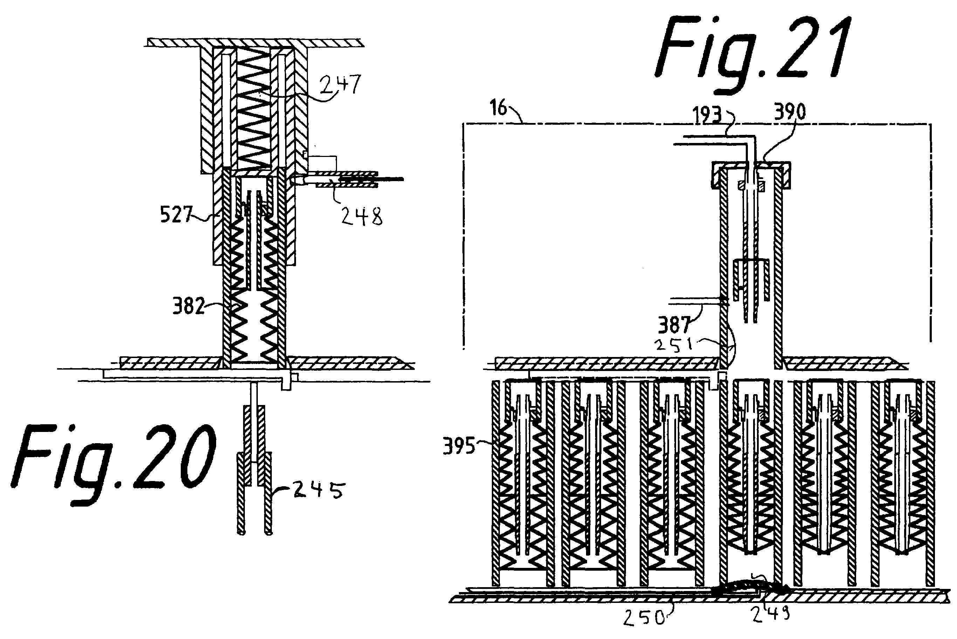

Die Figur 16 zeigt in einem Längsschnitt ein Versorgungsgerät für einen Injektor bei einer Schnittführung unterhalb der abnehmbaren Deckplatte(283,Fig.4 unten) in etwa natürlicher Größe. Rechts ist in einem Längsschnitt im Maßstab 2 : 1 die Variante einer Behältereinheit innerhalb der Verpackungszylinder zu erkennen.
Die Schienen(519,vgl.Fig.4 unten) des Versorgungsgerätes dienen der Befestigung des Gehäuses(16,vgl.Fig.4 oben) des Injektors. Die Verpackungszylinder(395) können hier auch ständig benutzte Gerätebestandteile sein und sind durch ein Band gegenseitig zu einer Kette verbunden. Letztere wird über die vier Spulen(714) zwischen den beiden Andruckkulissenleisten(520) geleitet und wird durch die Rollen(521) an Blattfedern gebremst, von denen wenigstens eine, gegen ihre Blattfeder zurückgebogen eine Kettenverlängerung zuläßt, so daß deren Wechsel ermöglicht wird. Die Mitnehmerklinke(522) dreht gegen die Druckfeder auf dem Schlitten(523) bei Bewegungsbegrenzung durch einen Anschlagstift(schwarz). Der Schlitten läuft auf einer Schiene (gestrichelt) mit seiner Querplatte (530) gegen die Druckfeder(524). Die Querplatte wird bei ihrer Verschiebung durch die vorspringende Deckelleiste (526,Fig.19 oben) über die sanfte Schräge des Rasters (525) bis hinter dessen Steilkante bewegt, wo sie festgehalten wird. Die Mitnehmeklinke hat dabei den nächsten Verpackungszylinder unter Sektordrehung um ihre Achse überholt. Bei Rückzug des Rasters(525) in die ihn umgebende Kanthülse gegen eine (nicht dargestellte) Druckfeder mittels eines (nicht dargestellten) Elektromagneten wird die Bewegung des Schlittens freigegeben und die Mitnehmerklinke verschiebt einen Verpackungszylinder nach links.
Die vergrößerte Variante einer Behältereinheit rechts zeigt Behälter für Verdünner(192) und Wasser(194) innerhalb der Unterteile(382) umgeben vom Oberteil(527) des Injektorschachtes(vgl.Fig.20). Die Austrittsstutzen(528) für die Flüssigkeiten sind zentral gelegen. Die Tülle (529) auf der Verbindungsplatte der Behältereinheit bildet die Verbindungsachse zur benachbarten Behältereinheit, von denen die rechte unterhalb in einer Bohrung liegt.
Durch die Tüllen verlaufen Stifte der Beförderungskette des Versorgungsgerätes (nicht dargestellt). Entlang dieser Stifte kann jede Behältereinheit etwa mittels einer Teleskopstange(245,Fig.20) in den Injektorschacht gehoben werden.

Die Figur 17 zeigt im Maßstab von 2 : 1 einen Vertikal- oder Querschnitt durch den oberen Teil der Behältereinheit der Fig.16 (rechts) mit einem Teil des aufnehmenden Injectorschachtes.
Die Austrittsstutzen(528) wurden in Bohrungen des Aufnahmedeckels(532) des genannten Schachtes eingeschoben und sind gegen denselben durch die zwei Dichtungsringe (195,246) abgedichtet. Der obere Raum, welcher durch diese Dichtungsringe abgedichtet ist, bildet eine Kammer, die durch einen Heizdraht(123) erhitzt werden kann, welcher seinerseits durch eine (nicht dargestellte) Batterie über Leitungen gespeist wird.

Die Figur 18 zeigt in einem Längsschnitt im Maßstab von ungefähr 3 : 1 eine Variante einer Behältereinheit.
Das Austrittsrohr(533) führt vom Faltenbalg, der als Behälter für Wasser(194) dient, aufwärts und ist gegen die innere Lippe des Aufnahmezylinders(534) des Injektors gedichtet. Der Endring(535) ist in eine Aushöhlung besagten Aufnahmezylinders mit einem weiteren Dichtungsring eingeschoben und dient der Ausstoßung des Verdünners aus dem Behälter(192) durch die Öffnung(536) zu einem (nicht dargestellten) Schlauch. Das Austrittsrohr(533) kann während der Entleerung des Wasserbehälters innerhalb der Innenbohrung des Aufnahmezylinders aufsteigen. Der Verdünnerauslaß befindet sich oben in Gestalt eines Schlauches.

Die Figur 19 zeigt, oben in einer Aufsicht von unten her einen Injektor. Der Schiebedeckel(537) befindet sich in seiner Führungsschiene (gestrichelt) mit vorspringenden Leiste, mit welcher der Deckel gegen seine Druckfeder geschoben und geöffnet wird. Zuvor stößt das Ende besagter Leiste(526), welche in einem Schlitz der Deckplatte des Injektors verläuft, den gefederten Hebel(539) aus dem Zick-Zack-Profil des Schiebedeckels, der dessen unbeabsichtigte Öffnung verhindert.
Unten wird ein schematischer Vertikal schnitt wiedergegeben, der die Schienenführung zwischen Injektor und Versorgungsgerät verdeutlicht.
Die Zeichnung wurde speziell zur Darstellung des Schiebedeckels(56,Fig.1) über der Saugglocke mit der Schiere(519) erweitert. Letztere weist ebenso wie die in die Schiene eingreifende Leiste des Injektors eine gummielastische Dichtleiste(202) auf, wobei die Dichtleisten durch leicht keilförmig sich verjüngenden Andruckleisten (203) an der Wandung(16) des Injektors in der Endphase des Schiebedeckel-Schlusses einander genähert werden.
Durch Beheizung des Saugglockenraumes kann so zwischen Benutzungen die Sterilität der Düsenumgebung auf eine solche der gesammten Saugglocke ausgedehnt werden. Die erhitzte Luft kann über das Rückschlagventil(43,Fig.1) entweichen.

Die Figur 20 zeigt in einem einen Längsschnitt in natürlicher Größe eine Behältereinheit nach Fig.18 in einem Injektorschacht. Es ist allein die Druckfeder(247) von der Innenausstattung des Oberteiles(527) dargestellt, welcher der Rückführung der verbrauchten Behältereinheit dient. Der Auslösestift(248) wird zur Rückführung der Behältereinheit (hier als Ganzes) in das Versorgungsgerät über das (nicht dargestellte) Steuergetriebe ausgelöst.
Unten wird die Teleskopstange(245) -zweckmäßigerweise als Teleleskopschraubhülsen vom Steuergetriebe her gedreht- gezeigt, welche die neue Behältereinheit in den Schacht des Injektors hebt, nachdem die trennenden Schiebedeckel von Injektor und Versorgungsgerät (letzterer nicht dargestellt) geöffnet wurden.

Die Figur 21 zeigt eine Reihe von Behälter-Einheiten in einem Versorgungsgerät in einem Längsschnitt in natürlicher Größe. Die drei linken Behälter-Einheiten sind unbenutzt, die drei rechten benutzt. Der Gummibogen(249) erlaubt durch Anhebung einer Behältereinheit unter dem Injektorschaft während des Daraufschiebens bei der Seitverschiebung der Behälter-Einheiten eine Abdichtung zwischen Behälter und Injektor. Der Transport der inneren Behältereinheit erfolgt durch Druckgas über den Kanal(250) durch eine Öffnung im Gummibogen(249), der als Kalotte zum Boden des Versorgungsgerätes gedichtet sein kann. Der Rücktransport der gebrauchten Behäler-Einheit erfolgt über Druckgaseinleitung über den Schlauch(387) an den Klemmfedern(251) vorbei.
Von den beiden Ableitungswegen für die Flüssigkeiten (vgl.Fig.18) ist das Abflußrohr(193) in der Deckelkappe (390) des Injektorschachtes abgebildet.

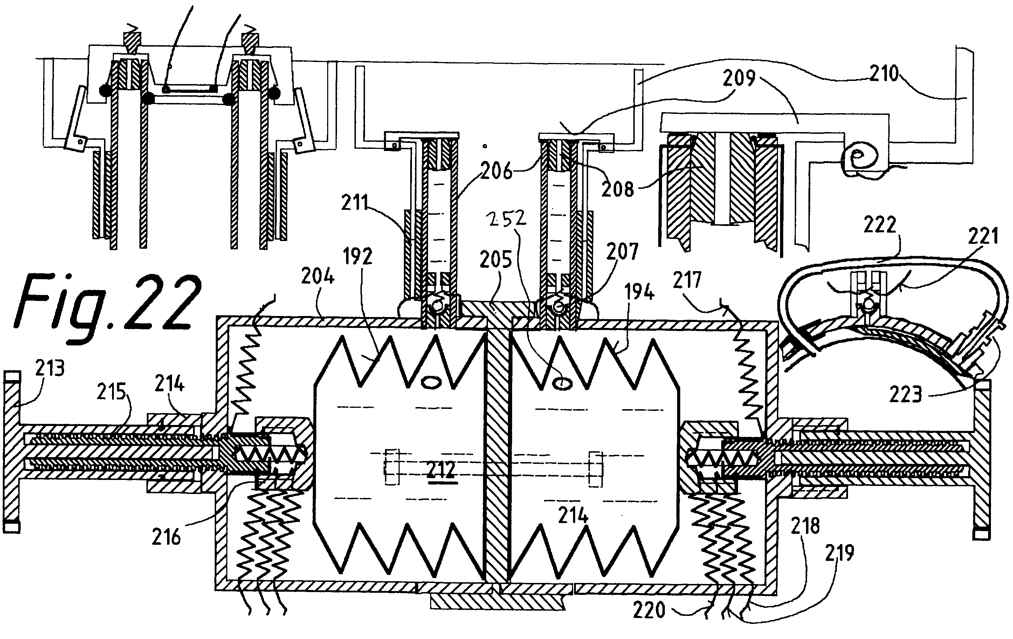

Die Figur 22 zeigt eine Abfüllvorrichtung für Flüssigkeiten aus großen Vorratsbehältern in einem Versorgungsgerät in einem Längsschnitt (unten) in natürlicher Größe.
Zwischen die beiden Gehäusezylinder(204) mit je einem Flatenbalg als Behälter für Verdünner(192) und Wasser (194) ist die Verbindungsmuffe(205) eingeschoben, welche als zentrales Drucklager für die Faltenbälge dient.
Innerhalb der seitlichen Tülle(214) ist das Artriebsrad -vom Steuergetriebe her betätigt- mit seiner Hülse drehbar. Ein von der Tülle ausgehender Stift greift dabei in eine Ringnut der Hülse. Letztere bewegt über ihr Innengewinde den Hohlbolzen(215), dessen Kopfbereich im Gehäusezylinder drehgesichert ist und innerhalb einer Mulde der Aufnahmeschale(216) gegen eine Druckfeder seitliches Bewegungsspiel hat. Kontakte auf dem Kopf des Hohlbolzens schließen nach einander einen Stromkreis zu Kontakten innerhalb der Aufnahmeschale, welche zur (nicht dargestellten) elektronischen Steuerzentrale weitergemeldet werden. Weitere elektrische Kontakte melden den Schluß des jeweiligen Rückschlagventiles(207) im Ausflußstutzen(206). Die aufrollbaren Kontaktdrähte(217,218, 219,220) sind (rechtsseitig) eingezeichnet. Die durch die Klammern(212) verbundenen Gehäuszylinder lassen den Luftaustausch zwischen dem Raum um die Faltenbälge und der Außenluft zu. Von der Schlauchmündung(252) fließt die Flüssigkeit über den Verbindungsschlauch(222), der einen Wanddurchbruch im Gehäuszylinder gesteckt ist und mit dem Stecknippel(223) über einen Querkanal mit dem Ausflußstutzen in Verbindung steht (Detail rechts in der Mitte im Vertikalschnitt). Der Ausflußstutzen mündet in einen Stopfen mit der Austrittsdüse(208), dem die Deckelklappe(209) aufliegt. Geöffnet werden die beiden Deckelklappen durch je eine Gabel(210), deren den Ausflußstutzen an liegender Teil jeweils im Gabelführungsstutzen(211) gegen eine Torsionsfeder um die Achse der Deckelklappe (als Rückführungskraft) verschieblich ist. Der Spalt zwischen Dichtung des Ausflußstutzens Deckelklappe und Stopfen mit Düse ist über einen elektrischen Heizdraht beheizt. Die Heizkammer kann auch durch eine ringförmig um beide Enden der Ausflußstutzen herumlaufende Kammer vergrößert werden. (*Rechts oben im Maßstab 3 : 1 im Detail)
Das Detail links oben zeigt den Zustand nach Öffnung der Deckelklappen durch die Absenkung der Gabelenden bei deren Anstoßen gegen die Injektorwand. (Eine zusätzliche Druckfeder zur späteren Rückführung der Gabeln wurde weggelassen). Die Ausflußstutzen sind in den Dichtungsbereich des Aufnahmedeckels eingedrungen (vgl.Fig.17).

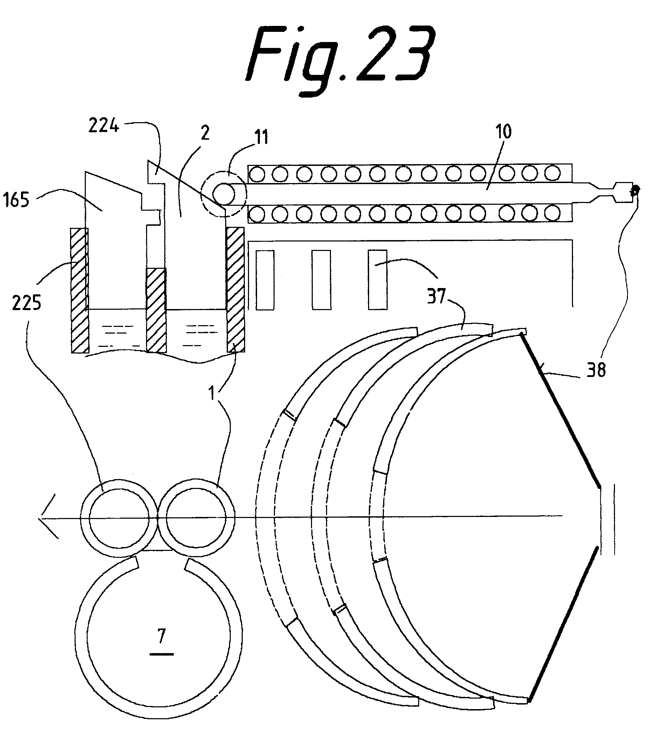

Die Figur 23 demonstriert in schematischem Querschnitt in natürlicher Größe oaben die Einwirkung eines einzigen Druckgebers in der Form des Kantstiftes(10) mit der Endrolle(11) nacheinander auf die Keilschräge der Injektionszylinderkolben(2,165). Dabei bewirkt die Randnoppe (224) des Injektionskolbens(2), daß noch in der Endphase seiner Absenkung auch der Injektionskolben(165) seine Abwärtsbewegung beginnt. Das Bewegungsmoment entsteht unter der Wirkung der gespannten Stahlfedern(37) über die Bogensehne(38). Im Längsschnitt unten gibt der Pfeil die Bewegungsrichtung des Kantstiftes als Druckgeber an.
Auch ist die Lagebeziehung der Injektionszylinder(1,225) zur Saugglocke(7) zu erkennen. Der Auslösemechanismus wurde zu Fig.1 beschrieben.

Die Figur 24 gibt ein mechanisches Steuergetriebe im Längsschnitt ungefähr in natürlicher Größe wieder.
(Es sind allerdings noch erhebliche Verkleinerungen bei Wahl der Zahnräder und am Kolben der Schubstange möglich, da nur geringe Kräfte benötigt werden).
Von der Motorachse(25) läuft der Antrieb über die Übersetzungszahnräder(98) auf die Vierkantachse(627), auf welcher das Zahnrad(45) verschieblich über die Haltelaschen(233) mit dem Ritzel(46) in Verbindung steht.

(Vgl.unten in einem Vertikalschnitt längs der Schnittlinie A - B. Von den Sperrzahnrädern für den Antrieb der beiden Miksrometerschrauben für die Dosierung (vgl. 106,Fig.9)ist nur das eine(631) im Längsschnitt zu erkennen. Wenn das Ritzel(46) mit seinen seitlichen Noppen (48) auf solche der Zahnräder(802,803) aufläuft, dann werden diese in der jeweiligen Ritzeldrehrichtung mitgedreht. Für die Mitnahme des Schlittens(49) an dessen elastischer Zunge längs einer Kantstange zur Betätigung des Bowden-Kabels(50,vgl.Fig.2) über das Gestänge(996) wird nach jeder Pendelverschiebung des Ritzels(46) auf seiner Schraubspindel mittels des Schub-Torsions-Gliedes (180) mit der Mantelhülse infolge Stops am Anschlag(234) dessen Querstab in eine Ringnut im Kopf des Gestänges (996) eingeschwenkt und kann dies bis zur erneuten Umschaltung durch Anstoß am Anschlag verschieben.
Auf der Pendelbewegung des Ritzels zur Umschaltung auf die Arzneidosierung kann die Endphase zur Umschaltung des Schub-Torsions-Gliedes ausgelassen werden. (Die Anordnung des Schub-Torsions-Gliedes kann dann der Lösung in Fig.25 entsprechen). In den Endphasen der Ritzelverschiebung kommt mindestens eine von dessen seitlichen Noppen in Kontakt mit einer seitlichen Noppe des Zahrrades(802 oder (03), welche beide fest mit der Verschiespindel verbunden sind. Bis zur Bewegungumkehr können die genannten Zahnräder beliebig oft gedreht werden, übertragen jedoch ihr Bewegungsmoment über die Sperrzahnräder jeweils nur in einer Richtung. Zur Umschaltung der Wirkrichtung (etwa für das Zurückfahren der Mikrometerschauben aus den Arzneipatronen) können am Schiebeschalter(707) die Sperrzahnräder gefedert richtungsgeändert werden, wie es nur rechtsseitig dargestellt wurde, aber auch für das Sperrzahnrad(631) gültig ist (Vgl.auch den analogen Mechanismus zu Fig.9).
Eine Automatisierung der Wirkrichtungsumschaltung über Koppelung an die Schiebebewegung unter Mitwirkung des Querstabes des Schub-Torsions-Gliedes in einer anderen (zusätzlichen) Radiärstellung ist möglich. Wegen der geringen Reibung bei der Dosierung ist eine beispielsweise gegen das Zahnrad(803) gerichtete Bremsfeder(998) zweckmäßig, was für alle entsprechenden Problemlösungen (auch gilt.

Die Figur 25 zeigt in einem schematischen Längsschnitt etwa im Maßstab 2 : 1 eine andere Lösung für das mechanische Steuergetriebe. Für die Arzneidosierung ist ein Getriebe analog zu Fig.9 oben abgebildet. Gegen die (in einer Aufrollung dargestellten Räder mit Keilprofil(241) ist jeweils ein rückgefederter Schieber(240) gerichtet, dessen Plattfeder vom den Wellen der Profilleisten mitgenommen werden. Wegen der geringen Reibung im Bereich der Mikrometerschraube für die Dosierung ist es zweckmäßig außer für die beiden Keilprofilräder, sondern auch für das gemeinsam sozusagen im Freilauf angetriebene Zwischenzahnrad(108) oder für die Mikrometerschraube ein Rad(144) mit Rasterung vorzusehen. (Dies gilt auch für das Beispiel der Fig.9).
IN der Mitte wird das Ritzel(48) über die Rotation der Gewindespindel oder Schraube(47) seitwärts bewegt und nimmt über die Gabel(253) mit oberem(238) und unterem (235) Kugelschnäpper(Detail rechts im Vertikalschnitt) jeweils eine der beiden übereinander liegenden Profilleisten(236,237, unten im Maßstab 1 : 4 und auseinandergelegt) mit, indem der Kugelschnäpper(235) bei Rechtsverschiebung seine Delle in der Profilleiste(237) verläßt und die obere Profilleiste(236) nach Einrasten des Kugelschnäppers(238) in die Delle mitnimmt. (Und umgekehrt bei Linksverschiebung). Von jedem Wellengipfel der Profilleiste wird ein gefederter Schieber in dessen Wirkrichtung mitgenommen, um unter Federwirkung im Weltal in die Ausgangsstellung außerhalb des Keilprofilrades zurückzukehren. Die Betätigung des Schub-Torsions-Gliedes(180) erfolgt durch das Ritzel an der Blattfeder (239), die in beiden Richtungen auch überholt werden kann.

Während der seitlichen Ritzelverschiebung können nicht nur streckenmäßig festgelegte Arbeitsschübe über eine Gabel(253,Fig.25) abgegriffen werden. Das Ritzel kann während seiner Verschiebung auch an Arbeitszahnrädern vorbeigeführt werden. Letztere können nach Ankupplung über achsenseitliche Federung im Zahneingriff vom Ritzel angetrieben werden. Die Zahl der Arbeitsumdrehungen kann durch die Breite der Arbeitsräder bestimmt werden.

Die Figur 26 ist ein Längsschnitt durch einen Injektor in natürlicher Größe. Innerhalb eines strichpunktiert angedeuteten Gehäuses ist als Druckgeber ein Austausch-Druckgasbehälter(178,Vgl.Fig.15) eingeschoben, der den Kolben(104) der Arzneipatrone(103) antreibt. Ist diese als Einzeldosis vorgesehen, so kann das an einem Schraubbolzen längenverstellbare Kopplungsstück(254) entfernt und der Austausch-Druckgasbehälter direkt dem Mantel(255) für die Aufnahme der Arzneipatrone mitteles des Bajonettverschlusses(902) gegen den Dichtungsring(901) in der (gestrichelt angedeuteten) Halterung (256) angekoppelt werden. Der Faltenbalg(816) mit Verdünner vor dem Kolben(104) wird nach Austreibung der

Arznei vom Druckgeaber gegen eine Schneidkante hinter der Düse(5) gepreßt. Die Schneidkante wirkt gegen eine Sollbruchstelle oder Wandverdünnung im kleinen Faltenbalg(816) und sein Inhalt wird dem Injektionsstrahl hinzugefügt. Bei Nutzung des Koppelungsstückes kann die Arzneipatrone als Stufenspritze eingesetzt werden. Die Höhe der Einzeldosis wird am Schraubbolzen eingestellt.
Auf den kleinen Faltenbalg mit Verdünner muß dann verzichtet werden.
Der Unterdruck in der Saugglocke wird durch Rückzug des Kolbens(257) einer handelsüblichen Injektionsspritze (258) von Hand hergestellt. Der Sogaufbau innerhalb der Saugglocke kann über entsprechende Kontakte am Fenster (64) für die optische Hautüberprüfung, durch Änderung der Meßergebnisse dort oder an anderen bewegten Teilen der Steuereinheit (27) gemeldet und von dort ein Signal für die Beheizung des Öffnungsmechanismus des Austausch-Druckgasbehälters ausgelöst werden. Die elektronische Steuerzentrale(27) aktiviert hierfür den Stromfluß von der Batteeie(26).

Die Figur 27 ist ein schematischer Längsschnitt durch einen Injektor für Pulverinjektion im Maßstab 2 : 1.
Der Faltenbalg(259) mit dem Arzneipulvervorrat innerhalb des Behälters(261) mit der Druckfeder(260) zu dessen Entleerung wurde deutlich verkleinert wiedergegeben; ebenso der Zugstab(262) zum Rückzug der Zugfeder während der Faltenbalgmontage und zur Verbrauchskontrolle.
Das Pulver wird durch ein Rohr in die Bohrung der Antriebswelle für die Trommel des Pulverdosiers(263) eingefüllt. In der Trommel wird bei deren Drehung das Pulver in einer Spiralnut in das Innere des Injektionszylinders(1) entleert. Die Pulvermenge ist von der Anzahl der Trommeldrehungen abhängig und erfolgt über ein Dosiergetriebe entsprechend demjenigen der Fig.9, aber ohne den Einsatz eines Sperrzahnrades. Als Druckgeber dient wieder ein Austausch-Druckgasbehälter.
Anstelle des Hohlstößels(196,Fig.15) wird die Druckfeder durch das Stützkreuz(264) gehalten. Die Austausch-Gasdruckkapsel wird mittels der Klemmschrauben(266) gegen den Dichtungsring(265) gepreßt, um den Gasstrom in den (vorallem im Durchmesser überdimensionierten) Injektionszylinder zu kanalisieren. Zwischen Saugglocke und Düsenumgebung sowie am Ende des Mantels(255) zum Randanschlag des Injektionszylinders spannt sich die elastische Schutzmembran(168). Der Einwurfschlitz(267) für das Pulver im Injektionszylinder wird gegenüber der Öffnung zum Pulverdosierer durch Vorschub des Injektionszylinders mit den Klemmschrauben und dem Austausch-Druckgasbehälter in Richtung Saugglocke verlagert.
Während der Injektion preßt der Gasdruck die Trommel des Pulverdosierers gegen den Dichtungsring(268).
Die Trommel des Pulverdosierers ist rechts im Detail im Maßstab 4 : 1 herausgezeichnet, das Rad mit Rasterung(244) findet sich ganz rechts im Maßstab 2 : 1 .
Zur Injektions wird der Deckel(269) um seine Falznut mit welcher an der Druckfeder verschweißt ist in Richtung Injektionszylinder um geschlagen (v-förmig gestrichelt). Das mit dem Deckel(269) verbundene Druckfederende ist auch mit dem Innenstutzen verbunden.
Die Heizdrähte dienen der Abschmelzun der Ränder des Deckels(269). Eine die Sterilität bewahrende Abziehfolie über der Öffnung zum Injektionszylinder zur Lagerung ist für alle Druckgasbehälter erforderlich.
Die Sterilität im Injektionszylinder wird durch Beheizung nach jedem Gebrauch über den Heizdraht(123) gewährleistet. Die anschließende Düsenabdeckelung(vgl.Fig.5,10,11) wurde hier weggelassen.
Der Rechtsschutz der technischen Lösungen soll sich nicht nur auf die dargestellten Beispiele erstrecken, sondern auch auf Merkmalskombinationen auch unter Ersatz einzelner Funktionseinheiten (etwa solcher für die Sogerzeugung, die Dosierung u.a.) durch bekannte technischen Vorrichtungen. So kann der Stutzen am Austausch-Druckgasbehälter auch auf diesen außen aufgesetzt werden. Insbesondere kann die Hautfalte nicht nur durch Sog erzeugt werden, sondern auch durch Klemmbacken wie etwa in Euro 0 301 165 A2 (Fig.61). Beim Pulverdosierer ist Druckgas der der Arznei nachfolgende und den Punktionskanal reinigende Stoff.
Nachtrag zu Fig. 2: Die Zuleitungen für Insulin, Verdünner und Wasser können durch Rückschlagventile gesichert werden.
Zusatz zu Fig.1: Als vier gestrichelte Kreise sind die Rohrstutzen(85,Fig.7) eingezeichnet.

## Patentansprüche

1. Druckstrahlinjektor bei Anwendung gegenüber einer durch mechanische Vorrichtung faltenartig geformten Haut nebst Zubehör,
dadurch gekennzeichnet,
daß innerhalb einer Gehäuseeinheit wenigstens ein Behälter mit Arznei und wenigstens ein weiterer Behälter mit anderen Stoffen jeweils wenigstens in Verbindung mit einer Dosiervorrichtung jeweils mengenmäßig bestimmt wenigstens einem Injektionszylinder zugeführt werden,
wobei die Entleerung der Stoffe aus wenigstens einem Injektionszylinders mittels Verdrängung der jeweiligen Stoffmenge und Art wenigstens durch einer. Druckgeber durch eine gemeinsame Düse hindurch erfolgt,
und wobei die Stoffabfüllung in den jeweiligen Injektionszylinder und die Stoffaustreibung über wenigstens eine Ventilvorrichtung statthaben,
insofern auch die Düse allein als Ventilvorrichtung gewertet wird, und wobei wenigstens ein mechanisches Steuergetriebe vorzugsweise im Zusammenwirken mit einer elektronischen Steuereinheit, welche auch eine Programmierung erleichtert, mit elektrischer Energiequelle den Funktionsablauf regelt,
wobei Vorkehrungen zur Bewahrung der zu injizierenden Stoffe vor bakterieller Verunreinigung getroffen sind und vorzugsweise eine Vorrichtung zur optischen Überwachung der Injektionsstelle zur Vermeidung einer Punktion krankhafter Hautstellen vorhanden ist,
außerdem vorzugsweise eine Vorrichtung zur Nachfüllung von Behältern mit Stoffen, welche Vorrichtung, als Gehäuse vom Injektor getrennt,
selbst mit einer Dosiervorrichtung zur mengenmäßigen Bestimmung der im Injektor zu ergänzenden Stoffe ausgestattet ist.

2. Injektor nach Anspruch 1 ,
dadurch gekennzeichnet,
daß die Arznei in flüssiger Form vorliegt und daß Ventilvorrichtung so gestaltet ist, daß aus einem Behälter Wasser zur Reinigung zwischen den Einspritzungen von Arznei in benutzte Injektionszylinder geleitet und durch die Düse zur Reinigung ausgespritzt wird.

3. Injektor nach Anspruch 1 und 2 ,
dadurch gekennzeichnet,
daß eine Ventilvorrichtung so gestaltet ist, daß sie die Beimengung von Verdünnungsflüssigkeit zu einer Arznei in einem Injektionszylinder gestattet oder wenigstens dieser nachschichtet, so daß diese Stoffe durch die gemeinsame Düse durch wenigstens einen Druckgeber ausgestoßen werden.

4. Injektor nach Anspruch 1,
dadurch gekennzeichnet,
daß in wenigstens zeitweisem Kontakt mit der Düse eine Ventilvorrichtung vorhanden ist, welche über das mechanische Steuergetriebe vor der Austreibung von Stoffen die Düsenöffnung freigibt und vorzugsweise auch der Einleitung von Arznei und anderen Stoffen dient.

5. Injektor nach Anspruch 1,
dadurch gekennzeichnet,
daß zwei Injektionszylinder vorgesehen sind, von denen einer vor der Injektion unter die Haut nur mit Verdünnungsflüssigkeit zu einer die gegebenenfalls verdünnte Arznei ergänzenden Gesamtdosis gefüllt wird,
wobei wenigstens ein Druckgeber so gestaltet oder gesteuert ist, daß er einen beträchtlichen Anteil des Verdünners erst nach der Entleerung des Injektionszylinders mit der Arznei durch die Düse austreibt.

6. Injektor nach Anspruch 1 ,
dadurch gekennzeichnet,
wenigstens ein Stoff in wenigstens einem Behälter gelagert und über eine Ventilvorrichtung mit einem Injektionszylinder verbunden ist, wobei letzterer mit Arznei in Form eines dosierten Pulvers beschickt wird,
welches durch Druckgas, das aus einem gesonderten Behälter zur Injektion in den Injektionszylinder eingeleitet wird, über die Düse unter die Haut getrieben wird.

7. Injektor Zubehör nach Anspruch 1 ,
dadurch gekennzeichnet,
daß als ein Behälter für die Lagerung eines Stoffes ein Austausch-Druckgasbehälter dient, den ein röhrenförmiger Stutzen für einen Druckgeber, als welcher auch das Druckgas selbst dienen kann, angelagert ist,
welcher Stutzen abgedeckelt und mit verschließender Ringkarte versehen ist nach deren Abschmelzung unter Hitzeeinwirkung der Druckgeber aus dem Stutzen heraus wirksam wird, vorzugsweise nachdem eine Druckfeder innerhalb des Stutzens einen Deckel zum Innerer des Druckgasbehälters aufgestoßen hat, was durch Herstelung eines Druckausgleiches zwischen Druckgasbehälter und Stutzen über die Abschmelzung der Plombe in einer engeren Öffnung zwischen Druckgasbehälter und Stutzen ermöglicht wird.

8. Injektor Zubehör nach Anspruch 1 ,
dadurch gekennzeichnet,
daß je zwei elastische Behälter für die Lagerung von flüssigen Stoffen in einem gemeinsamen Verpackungszylinder zusammengefaßt werden, innerhalb dessen sie durch eine äußere Kraft, vorzugsweise durch Druckgas,
spätestens nach Verbringung in einen Injektorschacht unter Druck gesetzt werden, wobei für den getrennter Abfluß aus den Behältern an diesen jeweils eine zur Öffnung in eine Ableitungsleitung des Injektors bestimmte Dichtung vorhanden ist, und wobei der Verpackungszylinder vorzugsweise einen Kragen aufweist,
der durch Andruck gegen einen Dichtungsring im Injektorschacht zur Abtrennung zweier Gasddruckräume über der beider Behältern dienen kann.

9. Injektor nach Anspruch 1 ,
dadurch gekennzeichnet,
daß über einer Deckplatte des Gehäuses ein weiterer beweglicher Deckel vorgesehen ist, wobei zwischen einer größeren Oberfläche von Deckplatte und Deckel wenigstens ein Beutel gelagert ist, dessen größere Oberfläche einerseits mit der unbeweglichen Deckplatte des Gehäuses andererseits mit der dieser Deckplatte zugewandten Seite des Deckels fest, aber vorzugsweise lösbar, verbunden ist, und wobei zwischen Gehäuse und Deckel wenigstens eine Druckfeder vorgesehen ist, welche streckenweise in einem Stutzen verläuft und in zusammengepreßtem Zustand während der Annäherung des Deckels an die Deckplatte durch Verrasterung festgestellt weiden kann, und wobei jeder Beutel über eine Schlauchverbindung, die bei mehreren Beuteln auch eine gemeinsame sein kann, vorzugsweise über eine Ventilvorrichtung, in Verbindung mit einer Saugglocke als Vorrichtung zur Hautfaltenbildung steht, welche nach Auslösung der Druckfederverrasterung unter Sogwirkung auf die Haut bei Ausdehnung des jeweiligen Beutels wirksam wird, und vorzugsweise während der Zusammenpressung des jeweilig vorhandenen Beutels den enstehenden Luftüberdruck zur Zusammenpressung von elastischen Behältern oder sonstige Verkleinerung des Lagerraumes für Stoffe in ihrer Behältern nutzt und damit zum Stofftransport in Richtung des jeweiligen Injektionszylinders.

10. Injektor nach Anspruch 1 ,
dadurch gekennzeichnet,
daß die Strahlrichtung aus der Düse durch deren Anordnung im Injektionszylinder deutlich von der Senkrechten zu der den Injektor bei seiner Anwendung umgebenden Hautoberfläche abweicht und vorzugsweise Waagerecht und annähernd parallel zur zur umgebenden Hautoberfläche verläuft und die angehobene Hautfalte mehr an deren Basis durchdringt, wobei der Düsenstrahl in seinem Zentrum damit vorzugsweise längs der größten Gehäuseausdehnung und dessen Auflagefläche auf der Haut verläuft.

11. Injektor nach Anspruch 1 ,
dadurch gekennzeichnet,
daß zur optischen Hautüberprüfung auf Eignung für die Punktion durch den Düsenstrahl vor die Düse wenigstens zeitweise ein Fenster vorgelagert wird,
in welches die für die Punktion vorgesehene Hautstelle eindringen kann und in dessen Bereich eine Lichtmeßstrecke vorzugsweise tangential zum Fenster vorhanden ist, wobei bei Soganwendung als Mittel zur Hautfaltenbildung vorzugsweise zwischen Düsenumgebung und Fensterrahmen ein Spalt zur Entfaltung der Sogwirkung auf die Haut an der Punktionsstelle in Richtung Düse vorgesehen ist.
